(19) Europäisches Patentamt
European Patent Office
Office européen des brevets

(11) EP 1 539 747 B1

(12) **EUROPEAN PATENT SPECIFICATION**

(45) Date of publication and mention of the grant of the patent:
**02.11.2006 Bulletin 2006/44**

(21) Application number: **03797310.4**

(22) Date of filing: **17.09.2003**

(51) Int Cl.:
***C07D 417/12*** (2006.01) ***C07D 401/12*** (2006.01)
***C07D 403/12*** (2006.01) ***C07D 239/42*** (2006.01)
***C07D 213/73*** (2006.01) ***A61K 31/454*** (2006.01)
***A61K 31/4545*** (2006.01) ***A61K 31/501*** (2006.01)

(86) International application number:
**PCT/EP2003/010412**

(87) International publication number:
**WO 2004/026866 (01.04.2004 Gazette 2004/14)**

(54) **N-AROYL CYCLIC AMINES AS OREXIN RECEPTOR ANTAGONISTS**

CYCLISCHE N-AROYLAMINE ALS OREXINREZEPTORANTAGONISTEN

AMINES CYCLIQUES N-AROYLE UTILISEES COMME ANTAGONISTES DU RECEPTEUR D'OREXINE

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **18.09.2002 GB 0221691**
**18.09.2002 GB 0221690**

(43) Date of publication of application:
**15.06.2005 Bulletin 2005/24**

(73) Proprietor: **GLAXO GROUP LIMITED**
**Greenford,**
**Middlesex UB6 0NN (GB)**

(72) Inventors:
- **BRANCH, Clive Leslie,**
  **c/o GlaxoSmithKline**
  **Harlow,**
  **Essex CM19 5AW (GB)**
- **COULTON, Steven,**
  **c/o GlaxoSmithKline**
  **Harlow,**
  **Essex CM19 5AW (GB)**
- **JOHNS, Amanda,**
  **c/o GlaxoSmithKline**
  **Harlow,**
  **Essex CM19 5AW (GB)**
- **NASH, David John,**
  **c/o GlaxoSmithKline**
  **Harlow,**
  **Essex CM19 5AW (GB)**
- **PORTER, Roderick Alan,**
  **c/o GlaxoSmithKline**
  **Harlow,**
  **Essex CM19 56AW (GB)**
- **STEMP, Geoffrey,**
  **c/o GlaxoSmithKline**
  **Harlow,**
  **Essex CM19 5AW (GB)**

(74) Representative: **Lawrence, Geoffrey Mark Prouse**
**GlaxoSmithKline,**
**Corporate Intellectual Property,**
**980 Great West Road**
**Brentford,**
**Middlesex TW8 9GS (GB)**

(56) References cited:
**WO-A-01/96302** **WO-A-02/44172**
**WO-A-02/090355** **WO-A-03/051873**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Many medically significant biological processes are mediated by proteins participating in signal transduction pathways that involve G-proteins and/or second messengers.

Polypeptides and polynucleotides encoding the human 7-transmembrane G-protein coupled neuropeptide receptor, orexin-1 (HFGAN72), have been identified and are disclosed in EP-A-875565, EP-A-875566 and WO 96/34877. Polypeptides and polynucleotides encoding a second human orexin receptor, orexin-2 (HFGANP), have been identified and are disclosed in EP-A-893498.

Polypeptides and polynucleotides encoding polypeptides which are ligands for the orexin-1 receptor, e.g. orexin-A (Lig72A) are disclosed in EP-A-849361.

Orexin receptors are found in the mammalian host and may be responsible for many biological functions, including pathologies including, but not limited to, depression; anxiety; addictions; obsessive compulsive disorder; affective neurosis/disorder; depressive neurosis/disorder; anxiety neurosis; dysthymic disorder; behaviour disorder; mood disorder; sexual dysfunction; psychosexual dysfunction; sex disorder; sexual disorder; schizophrenia; manic depression; delerium; dementia; severe mental retardation and dyskinesias such as Huntington's disease and Gilles de la Tourett's syndrome; disturbed biological and circadian rhythms; feeding disorders, such as anorexia, bulimia, cachexia, and obesity; diabetes; appetite/taste disorders; vomiting/nausea; asthma; cancer; Parkinson's disease; Cushing's syndrome / disease; basophil adenoma; prolactinoma; hyperprolactinemia; hypopituitarism; hypophysis tumor / adenoma; hypothalamic diseases; Froehlich's syndrome; adrenohypophysis disease; hypophysis disease; hypophysis tumor / adenoma; pituitary growth hormone; adrenohypophysis hypofunction; adrenohypophysis hyperfunction; hypothalamic hypogonadism; Kallman's syndrome (anosmia, hyposmia); functional or psychogenic amenorrhea; hypopituitarism; hypothalamic hypothyroidism; hypothalamic-adrenal dysfunction; idiopathic hyperprolactinemia; hypothalamic disorders of growth hormone deficiency; idiopathic growth hormone deficiency; dwarfism; gigantism; acromegaly; disturbed biological and circadian rhythms; and sleep disturbances associated with such diseases as neurological disorders, neuropathic pain and restless leg syndrome, heart and lung diseases; acute and congestive heart failure; hypotension; hypertension; urinary retention; osteoporosis; angina pectoris; myocardial infarction; ischaemic or haemorrhagic stroke; subarachnoid haemorrhage; head injury such as sub-arachnoid haemorrhage associated with traumatic head injury; ulcers; allergies; benign prostatic hypertrophy; chronic renal failure; renal disease; impaired glucose tolerance; migraine; hyperalgesia; pain; enhanced or exaggerated sensitivity to pain, such as hyperalgesia, causalgia and allodynia; acute pain; bum pain; atypical facial pain; neuropathic pain; back pain; complex regional pain syndromes I and II; arthritic pain; sports injury pain; pain related to infection, e.g. HIV, post-polio syndrome, and post-herpetic neuralgia; phantom limb pain; labour pain; cancer pain; post-chemotherapy pain; post-stroke pain; post-operative pain; neuralgia; nausea and vomiting; conditions associated with visceral pain including irritable bowel syndrome, migraine and angina; urinary bladder incontinence e.g. urge incontinence; tolerance to narcotics or withdrawal from narcotics; sleep disorders; sleep apnea; narcolepsy; insomnia; parasomnia; jet-lag syndrome; and neurodegenerative disorders, which includes nosological entities such as disinhibition-dementia-parkinsonism-amyotrophy complex; pallido-ponto-nigral degeneration, epilepsy, and seizure disorders.

Experiments have shown that central administration of the ligand orexin-A (described in more detail below) stimulated food intake in freely-feeding rats during a 4 hour time period. This increase was approximately four-fold over control rats receiving vehicle. These data suggest that orexin-A may be an endogenous regulator of appetite. Therefore, antagonists of its receptor may be useful in the treatment of obesity and diabetes, see *Cell,* 1998, **92**, 573-585.

There is a significant incidence of obesity in westernised societies. According to WHO definitions a mean of 35% of subjects in 39 studies were overweight and a further 22% clinically obese. It has been estimated that 5.7% of all healthcare costs in the USA are a consequence of obesity. About 85% of Type 2 diabetics are obese, and diet and exercise are of value in all diabetics. The incidence of diagnosed diabetes in westernised countries is typically 5% and there are estimated to be an equal number undiagnosed. The incidence of both diseases is rising, demonstrating the inadequacy of current treatments which may be either ineffective or have toxicity risks including cardiovascular effects. Treatment of diabetes with sulfonylureas or insulin can cause hypoglycaemia, whilst metformin causes GI side-effects. No drug treatment for Type 2 diabetes has been shown to reduce the long-term complications of the disease. Insulin sensitisers will be useful for many diabetics, however they do not have an anti-obesity effect.

Rat sleep/EEG studies have also shown that central administration of orexin-A, an agonist of the orexin receptors, causes a dose-related increase in arousal, largely at the expense of a reduction in paradoxical sleep and slow wave sleep 2, when administered at the onset of the normal sleep period. Therefore antagonists of its receptor may be useful in the treatment of sleep disorders including insomnia.

The present invention provides N-aroyl cyclic amine derivatives which are non-peptide antagonists of human orexin receptors, in particular orexin-1 receptors. In particular, these compounds are of potential use in the treatment of obesity, including obesity observed in Type 2 (non-insulin-dependent) diabetes patients, and/or sleep disorders. Additionally these compounds are useful in the treatment of stroke, particularly ischemic or haemorrhagic stroke, and/or

blocking the emetic response, i.e. useful in the treatment of nausea and vomiting.

International Patent Applications WO99/09024, WO99/58533, WO00/47577 and WO00/47580 disclose phenyl urea derivatives and WO00/47576 discloses quinolinyl cinnamide derivatives as orexin receptor antagonists. WO01/96302 discloses N-aroyl cyclic amine derivatives.

According to the invention there is provided a compound of formula (I):

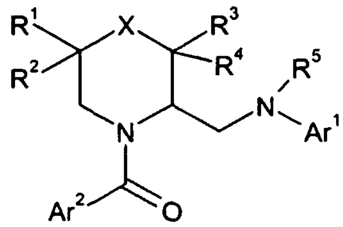

(I)

wherein:

X is O, $CR^7R^8$, NH or bond;
$R^1$ and $R^2$ are both hydrogen, both optionally substituted $(C_{1-4})$ alkyl, or are together with the carbon to which they are attached form a $(C_{3-6})$cycloalkyl ring or a 4- to 6- membered heterocyclyl ring.
$R^3$ and $R^4$ are both hydrogen, both optionally substituted $(C_{1-4})$ alkyl, or are together with the carbon to which they are attached form a $(C_{3-6})$cycloalkyl ring or a 4- to 6- membered heterocyclyl ring;
$R^7$ and $R^8$ are both hydrogen, both optionally substituted $(C_{1-4})$ alkyl, or are together with the carbon to which they are attached form a $(C_{3-6})$cycloalkyl ring or a 4- to 6- membered heterocyclyl ring;
provided that one pair of $R^1$ and $R^2$, $R^3$ and $R^4$, $R^7$ and $R^8$ are both optionally substituted $(C_{1-}$ 4) alkyl, or are together with the carbon to which they are attached form a $(C_{3-6})$cycloalkyl ring or a 4- to 6- membered heterocyclyl ring and the remaining groups are hydrogen;
$R^5$ is hydrogen, optionally substituted $(C_{1-4})$ alkyl, or optionally substituted $(C_{1-4})$alkylCO;
$Ar^1$ is an optionally substituted aryl, an optionally substituted mono or bicyclic heteroaryl group containing up to 3 heteroatoms selected from N, O and S;
$Ar^2$ represents phenyl or a 5- or 6-membered heterocyclyl group containing up to 3 heteroatoms selected from N, O and S, wherein the phenyl or heterocyclyl group is substituted by $R^6$ and further optional substituents; or $Ar^2$ represents an optionally substituted bicyclic aromatic or bicyclic heteroaromatic group containing up to 4 heteroatoms selected from N, O and S;
$R^6$ represents hydrogen, optionally substituted$(C_{1-4})$alkoxy, halo, cyano, optionally substituted$(C_{1-6})$alkyl, optionally substituted phenyl, or an optionally substituted 5- or 6-membered heterocyclyl group containing up to 4 heteroatoms selected from N, O and S;

or a pharmaceutically acceptable salt thereof.

When $R^1$ and $R^2$, $R^3$ and $R^4$ or $R^7$ and $R^8$ are optionally substituted $(C_{1-4})$ alkyl, the optionally substituted alkyl groups can be the same or different.

Preferably $R^7$ and $R^8$ if present are hydrogen.

Preferably $R^1$, $R^2$, $R^7$ and $R^8$ are hydrogen when $R^3$ and $R^4$ are methyl or $R^3$, $R^4$, $R^7$ and $R^8$ are hydrogen when $R^1$ and $R^2$ are methyl.

Preferably X is $CR^7R^8$

Preferably $R^5$ is hydrogen or optionally substituted $(C_{1-4})$ alkyl, more preferably hydrogen.

Preferably where $Ar^2$ represents phenyl or a 5- or 6-membered heterocyclyl group containing up to 3 heteroatoms selected from N, O and S, the $R^6$ group is situated adjacent to the point of attachment to the amide carbonyl.

$Ar^1$ may have up to 5, preferably 1, 2 or 3 optional substituents.

Examples of when $Ar^1$ is a mono or bicyclic heteroaryl group are quinoxalinyl, quinazolinyl, pyridopyrazinyl, benzoxazolyl, benzothiophenyl, benzimidazolyl, naphthyridinyl, pyridinyl, pyrimidinyl, thiazolyl, pyridazinyl, pyrazinyl, oxazolyl, triazolyl, imidazolyl, pyrazolyl, quinolinyl, benzofuranyl, indolyl, benzothiazolyl, oxazolyl[4,5-b]pyridinyl, pyridopyrimidinyl, isoquinolinyl, furanyl or thienyl.

Preferably $Ar^1$ is pyrimidinyl or pyridinyl.

When $Ar^2$ is a 5- or 6-membered heterocyclyl group containing up to 3 heteroatoms selected from N, O and S, it may be furanyl, thienyl, pyrrolyl, oxazolyl, thiazolyl, imidazolyl, oxadiazolyl, thiadiazolyl, pyridinyl, triazolyl, triazinyl,

pyridazinyl, pyrimidinyl, isothiazolyl, isoxazolyl, pyrazinyl or pyrazolyl.

When $Ar^2$ is an optionally substituted bicyclic aromatic or heteroaromatic it may be selected from benzofuranyl, benzimidazolyl, quinolinyl, quinoxalinyl, naphthyl, benzotriazolyl, benzothienyl, benzoxazolyl, naphthyridinyl, isoquinolinyl, quinazolinyl, indolyl, benzothiazolyl, or benzothiadiazolyl.

Preferably $Ar^2$ represents optionally substituted quinolinyl, thiazolyl, pyrazolyl, phenyl, naphthyl or quinoxalinyl.

When $R^6$ is a 5- or 6-membered heterocyclyl group containing up to 4 heteroatoms selected from N, O and S, it may be furanyl, thienyl, pyrrolyl, oxazolyl, thiazolyl, imidazolyl, oxadiazolyl, thiadiazolyl, pyridinyl, triazolyl, triazinyl, pyridazinyl, pyrimidinyl, isothiazolyl, isoxazolyl, pyrazinyl, pyrazolyl, tetrazolyl, piperazinyl, piperidinyl, morpholinyl, thiomorpholinyl or pyrrol indinyl.

Preferably when $R^6$ is a 5- or 6-membered heterocyclic ring containing up to 4 heteroatoms selected from N, O and S, it may be furanyl, thienyl, pyrrolyl, oxazolyl, thiazolyl, imidazolyl, oxadiazolyl, thiadiazolyl, pyridinyl, triazolyl, triazinyl, pyridazinyl, pyrimidinyl, isothiazolyl, isoxazolyl, pyrazinyl or pyrazolyl.

Preferably $R^6$ is selected from trifluoromethoxy, methoxy, ethoxy, halo, or an optionally substituted phenyl, pyridinyl, pyrazolyl, pyrimidinyl, or oxadiazolyl group.

When $R^1$ and $R^2$ or $R^3$ and $R^4$ or $R^7$ and $R^8$ together with the carbon to which they are attached form a 4- to 6- membered heterocyclyl ring, it may be selected from morpholinyl, piperidinyl, pyrrolidinyl, tetrahydrofuranyl, oxetanyl or azetidinyl.

Optional substituents for the groups $Ar^1$, $Ar^2$, and $R^6$ include halogen, hydroxy, oxo, cyano, nitro, $(C_{1-4})$alkyl, $(C_{1-4})$alkoxy, hydroxy$(C_{1-4})$alkyl, hydroxy$(C_{1-4})$alkoxy, halo$(C_{1-4})$alkyl, halo$(C_{1-4})$alkoxy, aryl$(C_{1-4})$alkoxy, $(C_{1-4})$alkylthio, hydroxy$(C_{1-4})$alkyl, $(C_{1-4})$alkoxy$(C_{1-4})$alkyl, $(C_{3-6})$cycloalkyl$(C_{1-4})$alkoxy, $(C_{1-4})$alkanoyl, $(C_{1-4})$alkoxycarbonyl, $(C_{1-4})$alkylsulfonyl, $(C_{1-4})$alkylsulfonyloxy, $(C_{1-4})$alkylsulfonyl$(C_{1-4})$alkyl, arylsulfonyl, arylsulfonyloxy, arylsulfonyl$(C_{1-4})$alkyl, $(C_{1-4})$alkylsulfonamido, $(C_{1-4})$alkylamido, $(C_{1-4})$alkylsulfonamido$(C_{1-4})$alkyl, $(C_{1-4})$alkylamido$(C_{1-4})$alkyl, arylsulfonamido, arylcarboxamido, arylsulfonamido$(C_{1-4})$alkyl, arylcarboxamido$(C_{1-4})$alkyl, aroyl, aroyl$(C_{1-4})$alkyl, or aryl$(C_{1-4})$alkanoyl group; a group $R^aR^bN$-, $R^aOCO(CH_2)_r$, $R^aCON(R^a)(CH_2)_r$, $R^aR^bNCO(CH_2)_r$, $R^aR^bNSO_2(CH_2)_r$ or $R^aSO_2NR^b(CH_2)_r$ where each of $R^a$ and $R^b$ independently represents a hydrogen atom or a $(C_{1-4})$alkyl group or where appropriate $R^aR^b$ forms part of a $(C_{3-6})$azacyloalkane or $(C_{3-6})$(2-oxo)azacycloalkane ring and r represents zero or an integer from 1 to 4, $(C_{1-4})$acyl, aryl, aryl$(C_{1-4})$alkyl, $(C_{1-4})$alkylamino$(C_{1-4})$alkyl, $R^aR^bN(CH_2)$n-, $R^aR^bN(CH_2)$nO-, wherein n represents an integer from 1 to 4, or when the substituent is $R^aR^bN(CH_2)$n- or $R^aR^bN(CH_2)$nO, $R^a$ with at least one $CH_2$ of the $(CH_2)$n portion of the group form a $(C_{3-6})$azacycloalkane and $R^b$ represents hydrogen, a $(C_{1-4})$alkyl group or with the nitrogen to which it is attached forms a second $(C_{3-6})$azacycloalkane fused to the first $(C_{3-6})$azacycloalkane.

Preferred optional substituents for $Ar^2$ are halogen, cyano, $(C_{1-4})$alkyl, or $(C_{1-4})$alkoxy.

Preferred optional substituents for $Ar^1$ are halogen. Most preferably the optional substituent for $Ar^1$ is bromine.

Preferred optional substituents for $R^6$ are halogen.

When $R^1$ to $R^4$, $R^7$ and $R^8$ are $(C_{1-4})$ alkyl the optionally substitutents can be halogen, hydroxy, $(C_{1-4})$alkoxy, hydroxy$(C_{1-4})$alkyl, hydroxy$(C_{1-4})$alkoxy, $(C_{1-4})$alkylthio, hydroxy$(C_{1-4})$alkyl, $(C_{1-4})$alkoxy$(C_{1-4})$alkyl or $(C_{3-6})$cycloalkyl$(C_{1-4})$alkoxy.

In the groups $Ar^1$ and $Ar^2$, substituents positioned *ortho* to one another may be linked to form a ring.

Preferred compounds of formula (I) are selected from:

(RS)-1-{2-[(5-Bromo-pyrimidin-2-ylamino)-methyl]-3,3-dimethyl-piperidin-1-yl}-1-[5-(4-fluorophenyl)-2-methyl-thiazol-4-yl]-methanone;
(RS)-1-{2-[(5-Bromo-pyrimidin-2-ylamino)-methyl]-3,3-dimethyl-piperidin-1-yl}-1-quinolin-8-yl-methanone;
1-{2-[(5-Bromo-pyrimidin-2-ylamino)-methyl]-3,3-dimethyl-piperidin-1-yl}-1-(2-methyl-quinolin-5-yl)-methanone;
(RS)-1-{2-[(5-Bromo-pyrimidin-2-ylamino)-methyl]-5,5-dimethyl-piperidin-1-yl}-1-(2-methyl-quinolin-5-yl)-methanone;
1-{2-[(5-Bromo-pyrimidin-2-ylamino)-methyl]-5,5-dimethyl-piperidin-1-yl}-1-(2-methyl-quinolin-5-yl)-methanone;
(RS)-1-{2-[(5-Bromo-pyridin-2-ylamino)-methyl]-3,3-dimethyl-piperidin-1-yl}-1-(2,3-dimethyl-quinoxalin-5-yl)-methanone;
(RS)-1 - {2-[(5-Bromo-pyridin-2-ylamino)-methyl]-3,3-dimethyl-piperidin-1-yl}-1-(2-methyl-quinolin-5-yl)-methanone; or
1-{(S)-2-[(5-Bromo-pyrimidin-2-ylamino)-methyl]-3,3-dimethyl-piperidin-1-yl}-1-(2,3-dimethyl-quinolin-8-yl)-methanone;

and pharmaceutically acceptable salts thereof.

When a halogen atom is present in the compound of formula (I) it may be fluorine, chlorine, bromine or iodine.

When the compound of formula (I) contains an alkyl group, whether alone or forming part of a larger group, e.g. alkoxy or alkylthio, the alkyl group may be straight chain, or branched or combinations thereof, it is preferably methyl or ethyl.

When used herein the term ($C_{4-6}$)cycloalkyl means a cycloalkyl group having 4, 5 or 6 carbon atoms, for instance cyclopropyl, cyclobutyl or cyclohexyl. Preferably it is cyclopropyl. Cycloalkyl groups can additionally be substituted by straight or branched alkyl groups.

When used herein the term aryl means a 5- to 6- membered aromatic ring for example phenyl, or a 7 to 12 membered bicyclic ring system where at least one of the rings is aromatic for example naphthyl.

It will be appreciated that compounds of formula (I) may exist as *R* or *S* enantiomers. The present invention includes within its scope all such isomers, including mixtures. Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses.

It will be understood that the invention includes pharmaceutically acceptable derivatives of compounds of formula (I) and that these are included within the scope of the invention.

Particular compounds according to the invention include those mentioned in the examples and their pharmaceutically acceptable derivatives.

As used herein "pharmaceutically acceptable derivative" includes any pharmaceutically acceptable salt, ester or salt of such ester of a compound of formula (I) which, upon administration to the recipient is capable of providing (directly or indirectly) a compound of formula (I) or an active metabolite or residue thereof.

It will be appreciated that for use in medicine the salts of the compounds of formula (I) should be pharmaceutically acceptable. Suitable pharmaceutically acceptable salts will be apparent to those skilled in the art and include acid addition salts formed with inorganic acids e.g. hydrochloric, hydrobromic, sulphuric, nitric or phosphoric acid; and organic acids e.g. succinic, maleic, acetic, fumaric, citric, tartaric, benzoic, p-toluenesulfonic, methanesulfonic or naphthalenesulfonic acid. Other salts e.g. oxalates, may be used, for example in the isolation of compounds of formula (I) and are included within the scope of this invention. Also included within the scope of the invention are solvates and hydrates of compounds of formula (**I**).

Certain of the compounds of formula (I) may form acid addition salts with one or more equivalents of the acid. The present invention includes within its scope all possible stoichiometric and non-stoichiometric forms.

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

According to a further feature of the invention there is provided a process for the preparation of compounds of formula (I) and derivatives thereof. The following schemes detail some synthetic routes to compounds of the invention.

**Scheme 1**

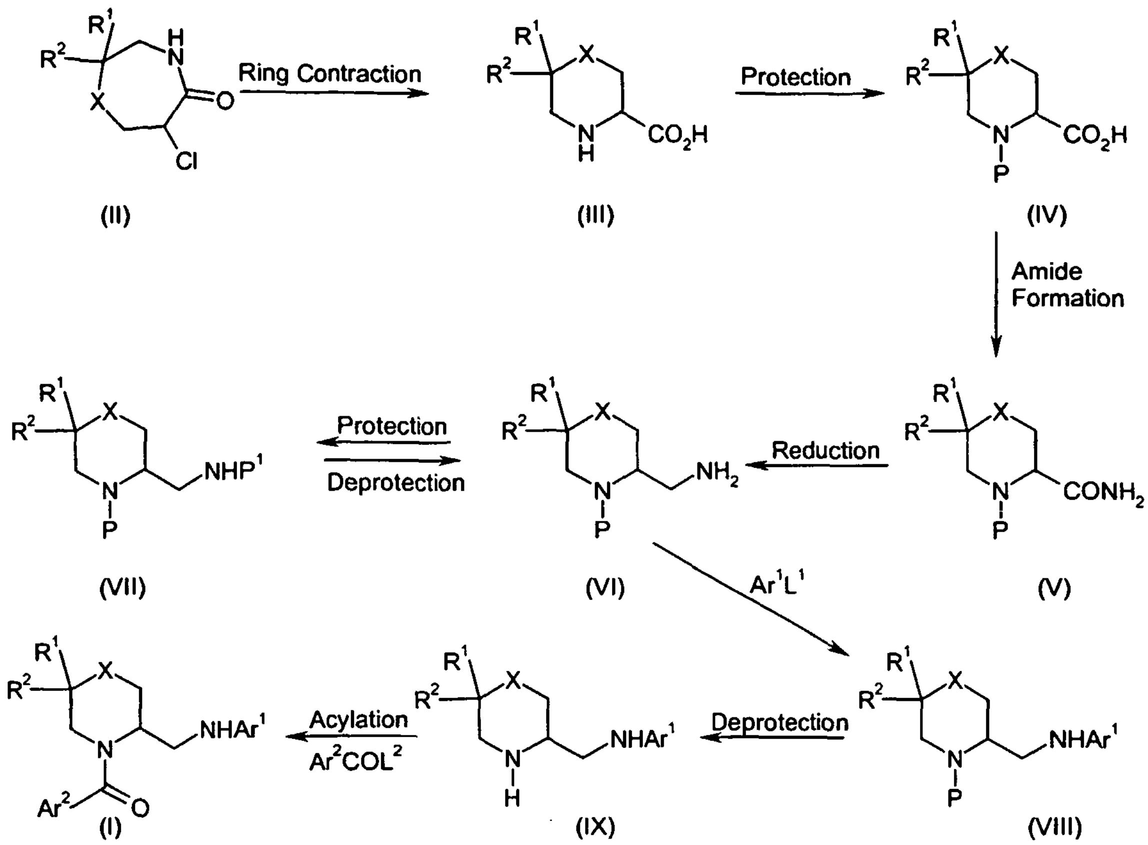


wherein X, $Ar^1$ and $Ar^2$ are as defined for formula (I), $R^1$ and $R^2$ are both optionally substituted $(C_{1-4})$alkyl, or are together with the carbon to which they are attached form a $(C_{3-6})$cycloalkyl ring or a 4- to 6- membered heterocyclyl ring, $L^1$ and $L^2$ are leaving groups, P and P' are protecting groups.

Examples of suitable leaving groups $L^1$ include halogen, $OSO_2Me$ and $OSO_2CF_3$. Reaction of amine (VI) to afford amine (VIII) proceeds in an inert solvent such as xylene, dimethylformamide, *N*-methylpyrrolidinone or a hydroxylic solvent such as t-butanol, in the presence of a base such as potassium carbonate, or diisopropylethylamine, preferably at elevated temperatures.

Examples of leaving groups $L^2$ include halogen, hydroxy, OC(=O)alkyl, OC(=O)O-alkyl and $OSO_2Me$. Acylation may be carried out using a wide range of conditions known in the literature, e.g. in an inert solvent such as dichloromethane, in the presence of a base such as triethylamine. Alternatively these steps may be carried out when $L^2$ represents hydroxy, in which case the reaction takes place in an inert solvent such as dichloromethane in the presence of a diimide reagent such as 1-ethyl-3-(3-dimethylaminopropyl)carbodiimide hydrochloride, and an activator such as 1-hydroxybenzotriazole or in dimethylformamide with *O*-(7-azabenzotriazol-1-yl)-*N,N,N'N'*-tetramethyluronium hexafluorophosphate

Examples of protecting groups P and $P^1$ include *t*-butyloxycarbonyl, trifluoroacetyl, benzyloxycarbonyl and optionally substituted benzyl. Deprotection conditions will depend on the particular protecting group; for the groups mentioned above these are respectively, acid (e.g. trifluoroacetic acid in dichloromethane), base (e.g. potassium carbonate in a solvent such as aqueous methanol) and catalytic hydrogenolysis in an inert solvent (e.g. using palladium on charcoal in a lower alcohol or ethyl acetate).

Compounds of formula (II) are known in the literature or can be prepared by known methods and converted into acids of type (II) using methods known in the art. For example, when $R^1$ and $R^2$ are both Me, and X is $CH_2$, according to EP 0447704 A1.

Reduction of the amide (V) to amine (VI) can be achieved using known methods e.g. by use of a metal hydride such as lithium aluminium hydride or borane, in an inert solvent such as tetrahydrofuran or diethyl ether.

Alkylation of compounds of formula (VII) to produce compounds where $R^5$ is optionally substituted alkyl, can be achieved using known methods e.g. by use of an alkylating agent such as methyl iodide in the presence of a metal hydride such as sodium hydride in a solvent such as dimethylformamide.

Compounds of formula (I) wherein $R^5$ is optionally substituted $(C_{1-4})$alkylCO can be made from compounds of formula (I) wherein $R^5$ is hydrogen by acylation reaction known in the literature.

Within the scope of this scheme, conversion of amine (VI) to amine (VIII) by reaction with a group $Ar^1L^1$ can

also be achieved without a protecting group P i.e. in compounds (VI) and (VIII) P can be H.

Included within the scope is protecting group interchange and use of optional protecting groups within $Ar^1$, $Ar^2$, $R^1$, $R^2$, $R^3$ , $R^4$ and X for example when X is NH, preferably a protecting group is used.

The synthetic route outlined in Scheme 1 can also be used for compounds (I) where $R^1$ and $R^2$ are H and X is $CH_2$, and $R^3$ and $R^4$ are as defined for formula (I), from compounds of formula (X) and (XI) which can be synthesised by methods known in the literature. For example when $R^3$ and $R^4$ are both Me, and X is $CH_2$ according to EP 0447704 A 1

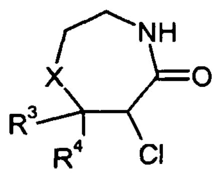

(X)

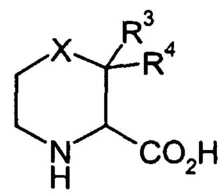

(XI)

or where $R^7$ and $R^8$ are both Me, according to EP 0447704 A1

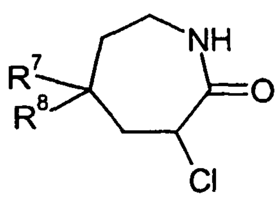

(XXIII)

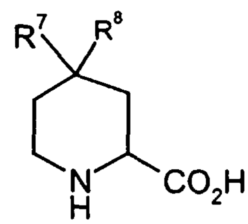

(XXIV)

Compounds of formula (XXIV) wherein $R^7$ and $R^8$ and the NH group is protected by a Boc group are disclosed in Castro et al, J. Med. Chem 1997, 40, 2491-2501.

Where X is a bond, compounds of the following formula are disclosed as follows:

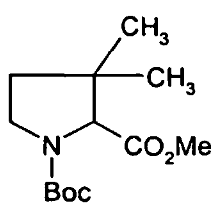

Sharma and Lubell
J.Org. Chem. 1996, 61,202-209

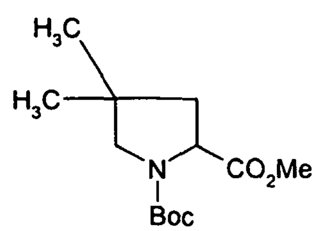

Soucy et al,
J. Chem. Soc. Perkin Trans 1,
1991, 11, 2885-2887

**Scheme 2**

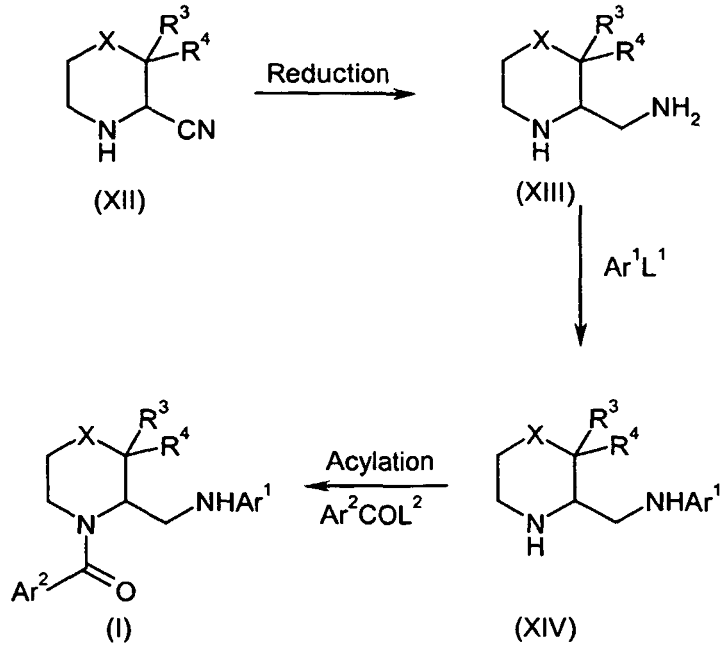


(XII) (XIII) (I) (XIV)

wherein X, $Ar^1$ and $Ar^2$ are as defined for formula (I), $R^3$ and $R^4$ are both optionally substituted $(C_{1-4})$alkyl, or are together with the carbon to which they are attached form a $(C_{3-6})$cycloalkyl ring or a 4- to 6- membered heterocyclyl ring and $L^1$ and $L^2$ are leaving groups and $P^1$ and $P^2$ are protecting groups as defined in Scheme 1.

Reduction of the nitrile (XII) to amine (XIII) can be achieved using known methods e.g. by the use of a metal hydride such as lithium aluminium hydride in an inert solvent such as tetrahydrofuran. Conversion of intermediates (XIII) to (XIV) and product (I) can be achieved as described for Scheme 1.

Compounds of formula (XII) can be synthesised using methods known in the literature. For example, when $R^3$ and $R^4$ are both Me and X is $CH_2$ , according to Martens *et al. J. Chem. Soc. Perkin Trans 1,* 2001, 508-13.

Included within the scope is protecting group interchange and use of optional protecting groups within $Ar^1$, $Ar^2$, $R^1$, $R^2$, $R^3$ , $R^4$ and X for example when X is NH, preferably a protecting group is used.

**Scheme 3**

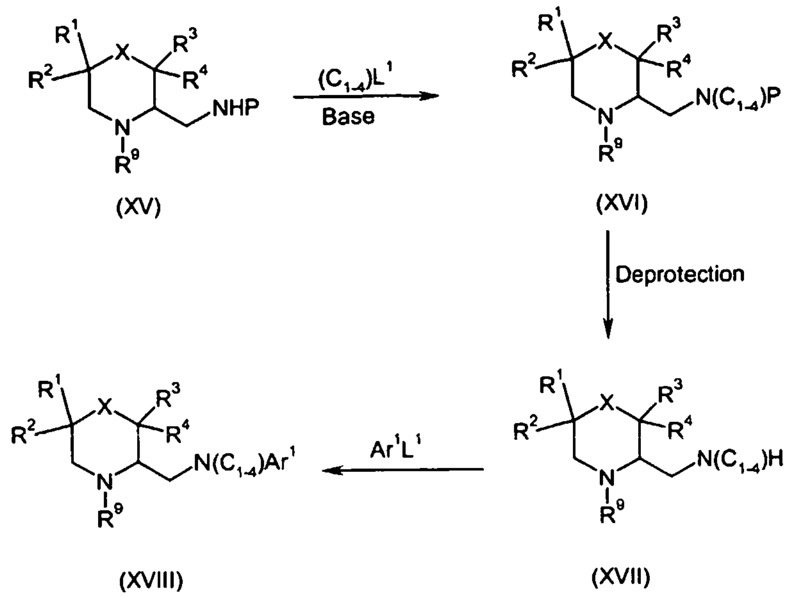


(XV) (XVI) (XVIII) (XVII)

wherein $R^1$, $R^2$, $R^3$, $R^4$, X and $Ar^1$ are as defined for formula (I) and $L^1$ is a leaving group and P a protecting group as defined for Scheme 1. $R^9$ can be either a protecting group $P^1$ or $Ar^2CO$ as defined for Scheme 1 and formula (I) respectively. For the conversion of (XVII) to (XVIII) $R^9$ can be H. In compounds of formula (XVIII) when $R^9$ is a protecting group $P^1$, deprotection gives (XVIII, $R^9$=H). Acylation of (XVIII) ($R^9$=H) with a group $Ar^2COL^2$ affords compounds of formula (I).

Reaction of (XV) with an alkylating agent $(C_{1-4})L^1$ proceeds in the presence of a base such as sodium hydride in an inert solvent such as dimethylformamide.

Included within the scope is protecting group interchange and use of optional protecting groups within $Ar^1$, $Ar^2$, $R^1$, $R^2$, $R^3$ , $R^4$ and X for example when X is NH, preferably a protecting group is used.

## Scheme 4

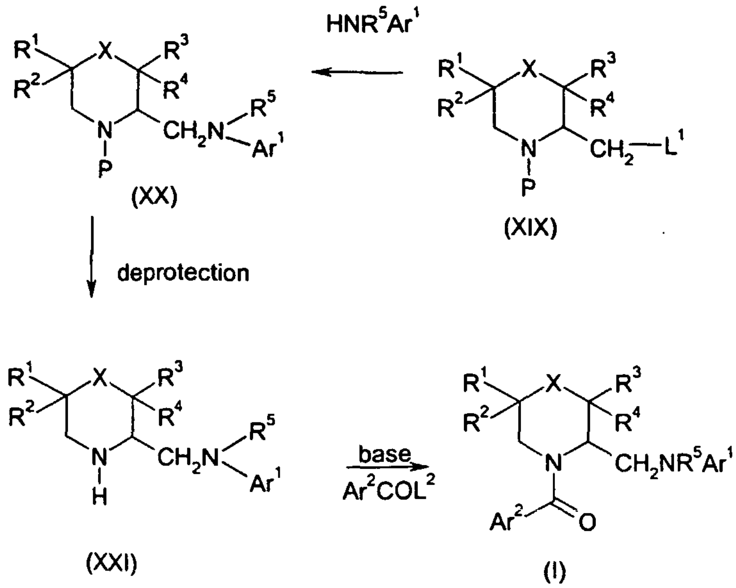

(XX) (XIX) (XXI) (I)

wherein $Ar^1$, $Ar^2$, $R^1$ to $R^5$ and X are as defined for formula (I), $L^1$ and $L^2$are leaving groups, and P is a protecting group.

Examples of suitable leaving groups $L^1$ include halogen, hydroxy, $OSO_2Me$, $OSO_2$(4-tolyl). The reaction of (XX) with $HNR^5Ar^1$ preferably proceeds in an inert solvent such as N,N-dimethylformamide in the presence of a base such as triethylamine, sodium hydride or potassium t-butoxide.

## Scheme 5

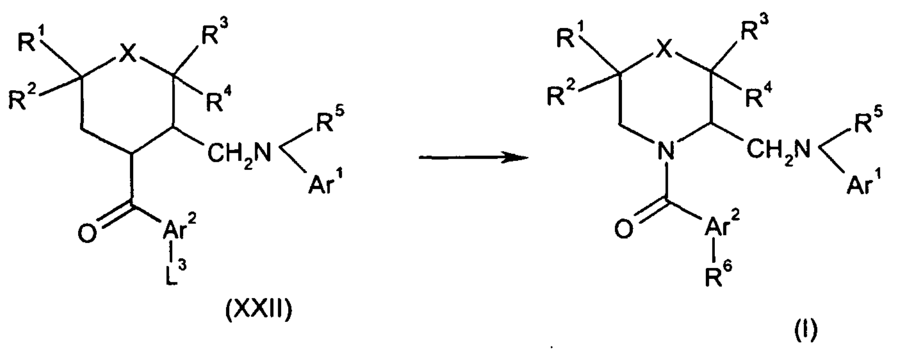
(XXII) (I)

wherein $Ar^1$, $Ar^2$, $R^1$ to $R^6$ and X are as defined for compounds of formula (I). $L^3$ is a leaving group.

The compounds of formula (I) may be prepared singly or as compound libraries comprising at least 2, e.g. 5 to 1000, preferably 10 to 100 compounds of formula (I). Compound libraries may be prepared by a combinatorial 'split and mix' approach or by multiple parallel synthesis using either solution phase or solid phase chemistry, by procedures

known to those skilled in the art.

Thus according to a further aspect of the invention there is provided a compound library comprising at least 2 compounds of formula (I), or pharmaceutically acceptable derivatives thereof.

Pharmaceutically acceptable salts may be prepared conventionally by reaction with the appropriate acid or acid derivative.

The compounds of formula (I) and their pharmaceutically acceptable derivatives are useful for the treatment of diseases or disorders where an antagonist of a human Orexin receptor is required such as obesity and diabetes; prolactinoma; hypoprolactinemia; hypothalamic disorders of growth hormone deficiency; idiopathic growth hormone deficiency; Cushings syndrome/disease; hypothalamic-adrenal dysfunction; dwarfism; sleep disorders; sleep apnea; narcolepsy; insomnia; parasomnia; jet-lag syndrome; sleep disturbances associated with diseases such as neurological disorders, neuropathic pain and restless leg syndrome; heart and lung diseases; depression; anxiety; addictions; obsessive compulsive disorder; affective neurosis/disorder; depressive neurosis/disorder; anxiety neurosis; dysthymic disorder; behaviour disorder; mood disorder; sexual dysfunction; psychosexual dysfunction; sex disorder; sexual disorder; schizophrenia; manic depression; delerium; dementia; bulimia and hypopituitarism. Additionally the compounds of formula (I) and pharmaceutically acceptable derivatives are useful for the treatment of stroke, particularly ischemic or haemorrhagic and/or in blocking an emetic response i.e. nausea and vomiting.

The compounds of formula (I) and their pharmaceutically acceptable derivatives are particularly useful for the treatment of obesity, including obesity associated with Type 2 diabetes, and sleep disorders. Additionally the compounds of formula (I) and pharmaceutically acceptable derivatives are useful for the treatment of stroke, particularly ischemic or haemorrhagic and/or in blocking an emetic response i.e. nausea and vomiting.

Other diseases or disorders which may be treated in accordance with the invention include disturbed biological and circadian rhythms; adrenohypophysis disease; hypophysis disease; hypophysis tumor / adenoma; adrenohypophysis hypofunction; functional or psychogenic amenorrhea; adrenohypophysis hyperfunction; migraine; hyperalgesia; pain; enhanced or exaggerated sensitivity to pain such as hyperalgesia, causalgia and allodynia; acute pain; burn pain; atypical facial pain; neuropathic pain; back pain; complex regional pain syndromes I and II; arthritic pain; sports injury pain; pain related to infection e.g. HIV, post-polio syndrome and post-herpetic neuralgia; phantom limb pain; labour pain; cancer pain; post-chemotherapy pain; post-stroke pain; post-operative pain; neuralgia; and tolerance to narcotics or withdrawal from narcotics.

The invention also provides the use of a compound of formula (I), or a pharmaceutically acceptable derivative thereof, in the manufacture of a medicament for the treatment or prophylaxis of diseases or disorders where an antagonist of a human Orexin receptor is required.

For use in therapy the compounds of the invention are usually administered as a pharmaceutical composition. The invention also provides a pharmaceutical composition comprising a compound of formula (I), or a pharmaceutically acceptable derivative thereof, and a pharmaceutically acceptable carrier.

The compounds of formula (I) and their pharmaceutically acceptable derivatives may be administered by any convenient method, e.g. by oral, parenteral, buccal, sublingual, nasal, rectal or transdermal administration, and the pharmaceutical compositions adapted accordingly.

The compounds of formula (I) and their pharmaceutically acceptable derivatives which are active when given orally can be formulated as liquids or solids, e.g. as syrups, suspensions, emulsions, tablets, capsules or lozenges.

A liquid formulation will generally consist of a suspension or solution of the active ingredient in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluorochloro-hydrocarbon or hydrofluorocarbon.

Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Preferably the composition is in unit dose form such as a tablet, capsule or ampoule.

The dose of the compound of formula (I), or a pharmaceutically acceptable derivative thereof, used in the treatment or prophylaxis of the abovementioned disorders or diseases will vary in the usual way with the particular disorder or disease being treated, the weight of the subject and other similar factors. However, as a general rule, suitable unit doses may be 0.05 to 1000 mg, more suitably 0.05 to 500 mg. Unit doses may be administered more than once a day for example two or three times a day, so that the total daily dosage is in the range of about 0.01 to 100 mg/kg; and such therapy may extend for a number of weeks or months. In the case of pharmaceutically acceptable derivatives the above figures are calculated as the parent compound of formula (I).

No toxicological effects are indicated/expected when a compound of formula (I) is administered in the above mentioned dosage range.

Human Orexin-A has the amino acid sequence:

pyroGlu Pro Leu Pro Asp Cys Cys Arg Gln Lys Thr Cys Ser Cys Arg Leu
1 5 10 15
Tyr Glu Leu Leu His Gly Ala Gly Asn His Ala Ala Gly Ile Leu Thr
20 25 30
Leu-$NH_2$

Orexin-A can be employed in screening procedures for compounds which inhibit the ligand's activation of the orexin-1 receptor.

In general, such screening procedures involve providing appropriate cells which express the orexin-1 receptor on their surface. Such cells include cells from mammals, yeast, Drosophila or *E. coli.* In particular, a polynucleotide encoding the orexin-1 receptor is used to transfect cells to express the receptor. The expressed receptor is then contacted with a test compound and an orexin-1 receptor ligand to observe inhibition of a functional response. One such screening procedure involves the use of melanophores which are transfected to express the orexin-1 receptor, as described in WO 92/01810.

Another screening procedure involves introducing RNA encoding the orexin-1 receptor into *Xenopus* oocytes to transiently express the receptor. The receptor oocytes are then contacted with a receptor ligand and a test compound, followed by detection of inhibition of a signal in the case of screening for compounds which are thought to inhibit activation of the receptor by the ligand.

Another method involves screening for compounds which inhibit activation of the receptor by determining inhibition of binding of a labelled orexin-1 receptor ligand to cells which have the receptor on their surface. This method involves transfecting a eukaryotic cell with DNA encoding the orexin-1 receptor such that the cell expresses the receptor on its surface and contacting the cell or cell membrane preparation with a compound in the presence of a labelled form of an orexin-1 receptor ligand. The ligand may contain a radioactive label. The amount of labelled ligand bound to the receptors is measured, e.g. by measuring radioactivity.

Yet another screening technique involves the use of FLIPR equipment for high throughput screening of test compounds that inhibit mobilisation of intracellular calcium ions, or other ions, by affecting the interaction of an orexin-1 receptor ligand with the orexin-1 receptor.

All publications, including but not limited to patents and patent applications, cited in this specification are herein incorporated by reference as if each individual publication were specifically and individually indicated to be incorporated by reference herein as though fully set forth.

The following Examples illustrate the preparation of pharmacologically active compounds of the invention. The Descriptions D1-D14 illustrate the preparation of intermediates to compounds of the invention.

**Description 1: (RS)-*C*-(3,3-Dimethyl-piperidin-2-yl)-methylamine**

A 1M solution of lithium aluminium hydride in tetrahydrofuran (114 ml) was added dropwise to a stirred solution of (RS)-3,3-dimethyl-piperidine-2-carbonitrile [Martens *et al, J. Chem. Soc., Perk Trans 1,* 2001, 508-13] (15.7 g, 0.114 mol) at room temperature under argon. The resultant mixture was stirred at room temperature for 0.5h then heated at

reflux for 1h. The mixture was cooled to room temperature and chilled in ice as water (3.5 ml), 20% sodium hydroxide (1.54 ml) and water (15 ml) were added dropwise sequentially with stirring. After 0.5h, anhydrous sodium sulphate was added, stirring continued for 0.5h and the mixture filtered and solids washed with diethyl ether. Combined filtrate and washings were evaporated *in vacuo* to afford the title compound as a pale orange oil (12.7g, 79%). Mass spectrum ($API^+$): Found 143 ($MH^+$). $C_8H_{18}N_2$ requires 142.

**Description 2: (RS)-(5-Bromo-pyrimidin-2-yl)-(3,3-dimethyl-piperidin-2-ylmethyl)-amine**

A mixture of (RS)-C-(3,3-dimethyl-piperidin-2-yl)-methylamine (D1) (7g, 0.049 mol), 5-bromo-2-chloropyrimidine (9.52g, 0.049 mol), diisopropylethylamine (26.4 ml, 0.147 mol) and potassium carbonate (13.6g, 0.099 mol) in xylene (250 ml) was heated at 120 ˚C under argon for 20h. On cooling the mixture was filtered, the filter cake washed with ethyl acetate and the combined filtrate and washings evaporated *in vacuo.* The residue was chromatographed on silica gel eluting with 0-10% methanol in ethyl acetate gradient then a 10% methanol in ethyl acetate mixture containing 2-4% 0.880 ammonia to afford the title compound as a pale orange solid (4.5g, 31%). Mass spectrum (Electrospray LC/MS): Found 299 ($MH^+$). $C_{12}H_{19}{}^{79}BrN_4$ requires 298.

The racemic product of Description 2 was separated into its individual enantiomers using the following procedure. Racemate (0.94g) was dissolved in ethanol to a concentration of 100mgml$^{-1}$. A 2 ml aliquot of this solution was applied to a Chiralpak AD (250 mm x 20 mm i.d.) chromatography column. Elution with ethanol at a flow rate of 17 mlmin$^{-1}$ using U.V. detection at 215 nm afforded the individual enantiomers. Repeat injection of 2 ml aliquots, pooling of relevant fractions and evaporation of the pooled fractions *in vacuo* afforded the following:-

**Description (2a):** Faster running enantiomer (0.29g). Mass spectrum (Electrospray LC/MS): Found 299 ($MH^+$). $C_{12}H_{19}{}^{79}BrN_4$ requires 298. Enantiomeric purity 99.9% e.e. $[\alpha]_D$ = +55.1˚ (c = 1, $CHCl_3$, at 29˚C)

**Description (2b):** Slower running enantiomer (0.29g). Mass spectrum (Electrospray LC/MS): Found 299 ($MH^+$). $C_{12}H_{19}{}^{79}BrN_4$ requires 298. Enantiomeric purity 99.9% e.e. $[\alpha]_D$ = -51.2˚ (c = 1, $CHCl_3$, at 28˚C)

**Description 3: (RS)-(5-Bromo-pyridin-2-yl)-(3,3-dimethyl-piperidin-2-ylmethyl)-amine**

A mixture of (RS)-*C*-(3,3-dimethyl-piperidin-2-yl)methylamine (D1) (0.2g, 1.41 mmol), 5-bromo-2-fluoro-pyridine (0.248g, 1.41 mmol), diisopropylethylamine (0.76 ml, 4.23 mmol) and potassium carbonate (0.389g, 2.82 mmol) in anhydrous dimethylformamide (5 ml) was heated at 100˚C under argon for 20h. The reaction mixture was cooled, evaporated *in vacuo* and the residue chromatographed on silica gel eluting with 0-10% methanol in ethyl acetate gradient then a 10% methanol in ethyl acetate mixture containing 2-4% 0.880 ammonia to afford the title compound as a pale orange oil (0.28g, 67%). Mass spectrum (Electrospray LC/MS): Found 298 ($MH^+$). $C_{13}H_{20}{}^{79}BrN_3$ requires 297.

The racemic product of Description 3 was separated into its individual enantiomers using the following procedure. Racemate (3.2g) was dissolved in ethanol to a concentration of 20mgml$^{-1}$. A 1 ml aliquot of this solution was applied to a Chiralpak AD (250 mm x 20 mm i.d.) chromatography column. Elution with ethanol at a flow rate of 17 mlmin$^{-1}$ using U.V. detection at 215 nm afforded the individual enantiomers. Repeat injection of 1 ml aliquots, pooling of relevant fractions and evaporation of the pooled fractions *in vacuo* afforded the following:-

**Description (3a):** Faster running enantiomer (0.45g). Mass spectrum (Electrospray LC/MS): Found 298 ($MH^+$). $C_{13}H_{20}{}^{79}BrN_3$ requires 297. Enantiomeric purity 96.4% e.e. $[\alpha]_D$ = +47.5˚ (c = 1, $CHCl_3$, at 30˚C)

**Description (3b):** Slower running enantiomer (0.45g). Mass spectrum (Electrospray LC/MS): Found 298 ($MH^+$). $C_{13}H_{20}{}^{79}BrN_3$ requires 297. Enantiomeric purity 94.8% e.e. $[\alpha]_D$ = -46.8˚ (c = 1, $CHCl_3$, at 28˚C)

**Description 4: (RS)-5,5-Dimethyl-piperidine-2-carboxylic acid**

A mixture of 3-chloro-6,6-dimethyl-azepan-2-one [EP0447704 A1] (18g, 0.103 mol) and barium hydroxide octahydrate (40.6g, 0.129 mol) in water (600 ml) was heated at reflux for 20h. On cooling, ammonium sulfate (17.13g, 0.129 mol) was added with stirring. The mixture was filtered through kieselguhr and the filtrate evaporated *in vacuo.* The residue was mixed with toluene and the mixture evaporated *in vacuo* to afford the title compound as a colourless solid (20g) which was used without further purification. $^1H$ NMR ($D_2O$) $\delta$ 1.00 (6H, m), 1.40 - 1.70 (2H, m), 1.75 - 1.90 (1H, m), 2.05 - 2.15 (1H, m), 2.75 - 2.85 (1H, m), 3.03 (1H, m), 3.40 - 3.55 (1H, m).

**Description 5: (RS)-5,5-Dimethyl-piperidine-1,2-dicarboxylic acid 1-*tert*-butyl ester**

To a solution of D4 (20g) and triethylamine (19.5 ml, 0.14 mol) in dioxan (800 ml) and water (140 ml) was added di-*t*-butyl dicarbonate (30.6g, 0.14 mol). The resultant mixture was stirred at room temperature for 48h. then evaporated *in vacuo.* The residue was partitioned between 1N sodium hydroxide and ethyl acetate (500 ml). The organic layer was extracted with 1N sodium hydroxide (2 x 100 ml). Combined aqueous layers were adjusted to pH6 with 5N hydrochloric acid and extracted with dichloromethane (3 x 250 ml). Combined extracts were dried ($Na_2SO_4$) and evaporated *in vacuo* to afford the title compound as a pale yellow gum (10.3g). NMR ($CDCl_3$) *inter alia* δ: 0.85 - 0.95 (6H, m), 1.10 - 1.30 (2H, m), 1.45 (9H, m), 1.87 (1H, m), 2.10 (1H, m), 2.75 - 2.95 (1H, m), 3.50 - 3.65 (1 H, m), 4.60 - 4.90 (1H, m), 7.20 - 8.90 (1H, br s).

**Description 6: (RS)-2-Carbamoyl-5,5-dimethyl-piperidine-1-carboxylic acid *tert*-butyl ester**

A mixture of (RS)-5,5-dimethyl-piperidine-1,2-dicarboxylic acid 1-*tert* butyl ester (D5) (9.5g, 37 mmol), 1-(3-dimethylaminopropyl)-3-ethylcarbodiimide hydrochloride (10.63g, 55 mol), 1-hydroxybenzotriazole (8.5g, 55 mmol), diisopropylethylamine (26 ml, 148 mmol) and ammonium chloride (3.96g, 74 mmol) in dimethylformamide (100 ml) was stirred at room temperature for 20h. then concentrated *in vacuo.* The residue was partitioned between water (500 ml) and ethyl acetate (500 ml) and the aqueous layer extracted with ethyl acetate (3 x 200 ml). Combined organics were washed with water (3 x 300 ml), saturated sodium hydrogen carbonate(300 ml), 0.5N hydrochloric acid (500 ml) and brine (250 ml), dried ($Na_2SO_4$) and evaporated *in vacuo.* The residue was chromatographed on silica gel eluting with 0-50% ethyl acetate in pentane gradient to afford the title compound as a colourless solid (5.3g, 54%). NMR ($CDCl_3$) δ: 0.88 (3H, s), 0.91 (3H, s), 1.20 - 1.50 (2H, m), 1.48 (9H, s), 1.75 (1H, m), 2.17 (1H, m), 2.57 (1H, br m), 3.40 - 3.85 (1H, br m), 4.83 (1H, br m), 5.58 (1H, br s), 6.06 (1H, br s).

**Description 7: (RS)-5,5-Dimethyl-piperidine-2-carboxylic acid amide**

A solution of (RS)-2-carbamoyl-5,5-dimethyl-piperidine-1-carboxylic acid *tert*-butyl ester (D6) (5.2g, 0.02 mol) in dichloromethane (50 ml) and trifluoroacetic acid (15 ml) was heated at 40˚C for 0.5h. The reaction mixture was evaporated *in vacuo* and the residue partitioned between dichloromethane (100 ml) and 1N sodium hydroxide (100 ml). The aqueous layer was extracted with dichloromethane (3 x 100 ml) and the combined organics dried ($Na_2SO_4$) and evaporated *in vacuo* to give the title compound as a colourless solid (3g, 95%). NMR ($CDCl_3$) δ: 0.89 (3H, s), 0.97 (3H, s), 1.20 - 1.35 (1H, m), 1.40 - 1.55 (1H, m), 1.60 - 1.75 (2H, m), 1.80 - 1.90 (1H, m), 2.45 (1H, m), 2.60 (1H, m), 3.15 (1H, m), 5.5 (1H, br s), 6.75 (1H, br s).

**Description 8: (RS)-1-Benzyl-5,5-dimethyl-piperidine-2-carboxylic acid amide**

A solution of (RS)-5,5-dimethyl-piperidine-2-carboxylic acid amide (D7) (3g, 19.2 mmol) and benzaldehyde (2.15 ml, 21.2 mmol) in 1,2-dichloroethane (120 ml) was stirred at room temperature for 1.5h under argon prior to addition of sodium triacetoxyborohydride (6.08g, 28.7 mmol) in one portion. The reaction mixture was stirred at room temperature for 24h, diluted with dichloromethane (120 ml) and washed with saturated sodium hydrogen carbonate. The organic layer was dried ($Na_2SO_4$) and evaporated *in vacuo.* The residue was chromatographed on silica gel eluting with 0-50% ethyl acetate in pentane gradient to afford the title compound as a colourless solid (4.24g, 90%). Mass spectrum ($API^+$): Found 247 ($MH^+$). $C_{15}H_{22}N_2O$ requires 246.

**Description 9: (RS)-*C*-(1-Benzyl-5,5-dimethyl-piperidin-2-yl)-methylamine**

A 1M solution of lithium aluminium hydride in tetrahydrofuran (20.7 ml) was added dropwise over 0.15h to a stirred solution of (RS)-1-benzyl-5,5-dimethyl-piperidine-2-carboxylic acid amide (D8) (4.22g, 17.2 mmol) and the resultant mixture stirred at room temperature for 0.5h then at reflux for a further 3h. On cooling, water (3.7ml), 2N sodium hydroxide (4.12 ml) and water (3.7 ml) were added dropwise sequentially followed after 0.1 h by anhydrous sodium sulphate. The mixture was filtered, the solids washed with tetrahydrofuran and the filtrates and washings combined and evaporated *in vacuo* to afford the title compound as a colourless solid (4g, 100 %). NMR ($CDCl_3$) δ: 0.79 (3H, s), 0.97 (3H, s), 1.10 - 1.60 (5H, m), 1.65 - 1.90 (2H, m), 2.15 (1H, m), 2.30 - 2.45 (1H, m), 2.65 - 2.75 (1H, m), 3.0 - 3.15 (2H, m), 4.07 (1H, m), 7.10 - 7.35 (5H, m).

**Description 10 : (RS)-*N*-(1-Benzyl-5,5-dimethyl-piperidin-2-ylmethyl)-2,2,2-trifluoroacetamide**

Trifluoroacetic anhydride (2.92 ml, 20.6 mmol) was added dropwise to a stirred solution of (RS)-C-(1-benzyl-5,5-dimethyl-piperidin-2-yl)-methylamine (D9) (3.98g, 17.2 mmol) and triethylamine (3.35 ml, 24 mmol) in anhydrous dichloromethane (100 ml) at 0˚C under argon. The resultant mixture was allowed to warm to room temperature, stirred for 20h, and washed with saturated sodium hydrogen carbonate. The organic layer was dried ($Na_2SO_4$) and evaporated *in vacuo.* The residue was chromatographed on silica gel eluting with 0-50% ethyl acetate in pentane gradient to yield the title compound as a colourless solid (3.85g, 66%). Mass spectrum (Electrospray LC/MS): Found 329 ($MH^+$). $C_{17}H_{23}F_3N_2O$ requires 328.

**Description 11: (RS)-5,5-Dimethyl-2-[(2,2,2-trifluoro-ethanoylamino)-methyl]-piperidine-1-carboxylic acid tert-butyl ester**

A solution of (RS)-*N*-(1-benzyl-5,5-dimethyl-piperidin-2-ylmethyl)-2,2,2-trifluoroacetamide (D10) (4.75g, 14.5 mmol) and di-*tert*-butyl dicarbonate (3.8g, 17.4 mmol) in ethanol (100 ml) was hydrogenated at atmospheric pressure and room temperature in the presence of 10% palladium on carbon (1g, 54% paste with water) for 70h. The mixture was filtered through kieselguhr, the filtrate evaporated *in vacuo* and the residue chromatographed on silica gel eluting with 0-10% ethyl acetate in pentane gradient to give the title compound as a colourless solid (4.57g, 93%). Mass spectrum ($API^+$): Found 339 ($MH^+$). $C_{15}H_{25}F_3N_2O_3$ requires 338.

**Description 12: (RS)-2-Aminomethyl-5,5-dimethyl-piperidine-1-carboxylic acid *tert* butyl ester**

A mixture of (RS)-5,5-dimethyl-2-[(2,2,2-trifluoro-ethanoylamino)-methyl]-piperidine-1-carboxylic acid *tert* butyl ester (D11) (4.56g, 12.6 mmol) and sodium carbonate (9.4g, 89 mmol) in methanol (300 ml) and water (100 ml) were heated at reflux for 2.5h under argon. Potassium carbonate (9.4g, 68 mmol) was added and reflux continued for 1h. The reaction mixture was cooled, evaporated *in vacuo* and the residue partitioned between water (100 ml) and dichloromethane (300 ml). The aqueous layer was extracted with dichloromethane (2 x 100 ml) and the combined organics dried ($Na_2SO_4$) and evaporated *in vacuo* to yield the title compound as a pale orange oil (3.2g, 98 %). NMR ($CDCl_3$) δ: 0.89 (3H, s), 0.91 (3H, s), 1.20 - 1.80 (5H, m), 1.46 (9H, s), 1.82 (1H, m), 2.50 (1H, m), 2.62 (1H, m), 2.90 (1H, m), 3.60 (1H, br s), 4.15 (1H, br s).

**Description 13: (RS)-2-[5-Bromo-pyrimidin-2-ylamino)-methyl]-5,5-dimethyl-piperidine-1-carboxylic acid *tert*-butyl ester**

A mixture of (RS)-2-aminomethyl-5,5-dimethyl-piperidine-1-carboxylic acid *tert*-butyl ester (2.7g, 11.2 mmol), 5-bromo-2-chloropyrimidine (2.04g, 11.2 mmol), potassium carbonate (3.1g, 22.5 mmol) and diisopropylethylamine (5.9 ml, 33.5 mmol) in xylene (100 ml) was heated at 130˚C for 20h under argon. The reaction mixture was cooled, filtered and the solid washed with ethyl acetate. Combined filtrates and washings were evaporated *in vacuo* and the residue chromatographed on silica gel eluting with 0-20% ethyl acetate in pentane gradient to give the title compound as a colourless solid (3.6g, 80%). NMR ($CDCl_3$) δ: 0.91 (6H, s), 1.27 (1H, m), 1.30 - 1.55 (2H, m), 1.40 (9H, s), 1.88 (1H, m), 2.59 (1H, m), 3.33 (1H, m), 3.40 - 3.75 (2H, m), 4.96 (1H, br s), 5.30 (1H, br s), 8.25 (2H, s).

**Description 14: (RS)-(5-Bromo-pyrimidin-2-yl)-(5,5-dimethyl-piperidin-2-ylmethyl)-amine**

The title compound was prepared from (RS)-2-[5-bromo-pyrimidin-2-ylamino)-methyl]-5,5-dimethyl-piperidin-1-carboxylic acid *tert*-butyl ester (D13) (3.6g, 9.0 mmol) using the method of description D7 as a pale orange solid (2.6g, 97%). NMR ($CDCl_3$) δ: 0.85 (3H, s), 0.98 (3H, s), 1.20 - 1.60 (5H, m), 2.42 (1H, m), 2.55 - 2.70 (2H, m), 3.25 (1H, m), 3.46 (1H, m), 5.64 (1H, br s), 8.26 (2H, s).

The racemic product of Description 14 was separated into its individual enantiomers using the following procedure. Racemate (2.5g) was dissolved in ethanol to a concentration of $200 mgml^{-1}$. A 1 ml aliquot of this solution was applied to a Chiralpak AD (200 mm x 50 mm i.d.) chromatography column. Elution with ethanol containing 0.1% triethylamine at a flow rate of $50 mlmin^{-1}$ using U.V. detection at 230 nm afforded the individual enantiomers. Repeat injection of 1 ml aliquots, pooling of relevant fractions and evaporation of the pooled fractions *in vacuo* afforded the following:-

**Description (14a):** Faster running enantiomer (1.07g). Mass spectrum (Electrospray LC/MS): Found 299 ($MH^+$). $C_{12}H_{19}{}^{79}BrN_4$ requires 298. Enantiomeric purity 99% e.e. $[\alpha]_D$ = -21.7° (c = 1, $CHCl_3$, at 29°C)

**Description (14b):** Slower running enantiomer (0.975g). Mass spectrum (Electrospray LC/MS): Found 299 ($MH^+$). $C_{12}H_{19}{}^{79}BrN_4$ requires 298. Enantiomeric purity 98% e.e. $[\alpha]_D$ = +16.5° (c = 1, $CHCl_3$, at 29°C)

**Example 1: (RS)-1-{2-[(5-Bromo-pyrimidin-2-ylamino)-methyl]-3,3-dimethyl-piperidin-1-yl}-1-[5-(4-fluoro-phenyl)-2-methyl-thiazol-4-yl]-methanone**

A solution of 5-(4-fluoro-phenyl)-2-methyl-thiazole-4-carbonyl chloride (0.094g, 0.37 mmol) in dichloromethane (1 ml) was added dropwise to a stirred solution of (RS)-5-bromo-pyrimidin-2-yl)-(3,3-dimethyl-piperidin-2-ylmethyl)-amine (D2) (0.1g, 0.33 mmol) and triethylamine (0.1 ml, 0.74 mmol) in dichloromethane (3 ml). After 2.5h the reaction mixture was washed with saturated sodium hydrogen carbonate (8ml) and the organic layer applied to a 10g silica gel column. Elution with 0-100% ethyl acetate in hexane gradient afforded the title compound as a colourless solid (0.135g, 78%). Mass spectrum (Electrospray LC/MS) Found 518 ($MH^+$). $C_{23}H_{25}{}^{79}BrFN_5OS$ requires 517.

**Example 2: (RS)-1-{2-[(5-Bromo-pyrimidin-2-ylamino)-methyl]-3,3-dimethyl-piperidin-1-yl}-1-quinolin-8-yl-methanone**

A mixture of (RS)-(5-bromo-pyrimidin-2-yl)-(3,3-dimethyl-piperidin-2-ylmethyl)-amine (D2) (0.08g, 0.27 mmol), quinoline-8-carboxylic acid (0.046g, 0.27 mmol), *O*-(7-azabenzotriazol-1-yl)-N,N,$N^1$,$N^1$-tetramethyluronium hexafluorophosphate (HATU) (0.102g, 0.27 mmol) and diisopropylethylamine (0.14 ml, 0.81 mmol) in anhydrous dimethylformamide (6 ml) was stirred at room temperature for 24h. The reaction mixture was evaporated *in vacuo* and the residue dissolved in ethyl acetate and washed with water. The organic layer was dried ($Na_2SO_4$) and evaporated *in vacuo.* The residue was chromatographed on silica gel using 0-100% ethyl acetate in pentane gradient then 0-10% methanol in ethyl acetate gradient to afford the title compound as a colourless solid (0.039g, 32%). Mass spectrum (Electrospray LC/MS): Found 454 ($MH^+$). $C_{22}H_{24}{}^{79}BrN_5O$ requires 453.

**Example 3: 1-{2-[(5-Bromo-pyrimidin-2-ylamino)-methyl]-3,3-dimethyl-piperidin-1-yl}-1-(2-methyl-quinolin-5-yl)-methanone**

A mixture of (+)-(5-bromo-pyrimidin-2-yl)-(3,3-dimethyl-piperidin-2-ylmethyl)-amine (D2a) (0.075g, 0.25 mmol), 2-methyl-quinoline-5-carboxylic acid (0.047g, 0.25mmol), HATU (0.096g, 0.25 mmol) and diisopropylethylamine (0.13ml, 0.75 mmol) in anhydrous dimethylformamide (3.5ml) was stirred at room temperature for 24h. The reaction mixture was evaporated *in vacuo* and the residue dissolved in ethyl acetate and washed with water. The organic layer was dried ($Na_2SO_4$) and evaporated *in vacuo.* The residue was chromatographed on silica gel using 0-100% ethyl acetate in pentane gradient then 0-10% methanol in ethyl acetate gradient to afford the title compound as a colourless solid (0.06g, 53%). Mass spectrum (Electrospray LC/MS): Found 468 ($MH^+$). $C_{23}H_{26}{}^{79}BrN_5O$ requires 467. The absolute stereochemistry of Example 3 is undefined.

**Example 4: (RS)-1-{2-[(5-Bromo-pyrimidin-2-ylamino)-methyl]-5,5-dimethyl-piperidin-1-yl}-1-(2-methyl-quinolin-5-yl)-methanone**

A mixture of (RS)-5-(bromo-pyrimidin-2-yl)-(5,5-dimethyl-piperidin-2-ylmethyl)-amine (D14) (0.1g, 0.33 mmol), 2-methyl-quinoline-5-carboxylic acid (0.068g, 0.36 mmol), HATU (0.125g, 0.33 mmol) and diisopropylethylamine (0.18ml, 0.99 mmol) in anhydrous dimethylformamide (5ml) was stirred at room temperature for 24h. The reaction mixture was evaporated *in vacuo* and the residue dissolved in ethyl acetate and washed with water. The organic layer was dried ($Na_2SO_4$) and evaporated *in vacuo.* The residue was chromatographed on silica gel using 0-100% ethyl acetate in pentane gradient then 0-10% methanol in ethyl acetate gradient to afford the title compound as a colourless solid (0.103g, 66%). Mass spectrum (Electrospray LC/MS): Found 468 ($MH^+$). $C_{23}H_{26}{}^{79}BrN_5O$ requires 467.

**Example 5: 1-{2-[(5-Bromo-pyrimidin-2-ylamino)-methyl]-5,5-dimethyl-piperidin-1-yl}-1-(2-methyl-quinolin-5-yl)-methanone**

A mixture of (+)-5-(bromo-pyrimidin-2-yl)-(5,5-dimethyl-piperidin-2-ylmethyl)-amine (D14b) (0.12g, 0.4 mmol), 2-methyl-quinoline-5-carboxylic acid (0.075g, 0.4 mmol), 1-[3-(dimethylamino)propyl]-3-ethylcarbodiimide hydrochloride (EDC) (0.077g, 0.4 mmol) and 1-hydroxybenzotriazole hydrate (HOBt) (0.01g, 0.07 mmol) in dichloromethane (4ml) was shaken at room temperature for 20h. The reaction mixture was washed with saturated aqueous sodium hydrogen

carbonate (8ml) and the organic layer applied to a pre-packed 10g silica column. Elution with 0-100% ethyl acetate in pentane gradient then 0-10% methanol in ethyl acetate gradient gave the title compound as a colourless solid (0.062g, 33%). Mass spectrum (Electrospray LC/MS): Found 468 ($MH^+$). $C_{23}H_{26}{}^{79}BrN_5O$ requires 467.

**Example 6: (RS)-1-{2-[(5-Bromo-pyridin-2-ylamino)-methyl]-3,3-dimethyl-piperidin-1-yl}-1-(2,3-dimethyl-quinoxalin-5-yl)-methanone**

A mixture of (RS)-(5-bromo-pyridin-2-yl)-(3,3-dimethyl-piperidin-2-ylmethyl)-amine (D3) (0.12g, 0.4 mmol), 2,3-dimethyl-quinoxaline-5-carboxylic acid (0.082g, 0.4 mmol), 1-[3-(dimethylamino)propyl]-3-ethylcarbodiimide hydrochloride (EDC) (0.077g, 0.4 mmol) and 1-hydroxybenzotriazole hydrate (HOBt) (0.01g, 0.07 mmol) in dichloromethane (4ml) was shaken at room temperature for 20h. The reaction mixture was washed with saturated aqueous sodium hydrogen carbonate (8ml) and the organic layer applied to a pre-packed 10g silica column. Elution with 0-100% ethyl acetate in pentane gradient then 0-10% methanol in ethyl acetate afforded a colourless solid. Mass directed purification on an $ABZ^+$ C8 (100 mm x 21 mm i.d.) chromatography column eluting with 0 - 95% acetonitrile in water containg 0.1% trifluoroacetic acid at a flow rate of 3 $mlmin^{-1}$ afforded the title compound as the trifluoroacetate salt (0.05g, 21%). Mass spectrum (Electrospray LC/MS): Found 482 ($MH^+$). $C_{24}H_{28}{}^{79}BrN_5O$ requires 481.

**Example 7: (RS)-1-{2-[(5-Bromo-pyridin-2-ylamino)-methyl]-3,3-dimethyl-piperidin-1-yl}-1-(2-methyl-quinolin-5-yl)-methanone**

A mixture of (RS)-(5-bromo-pyridin-2-yl)-(3,3-dimethyl-piperidin-2-ylmethyl)-amine (D3) (0.1g, 0.34 mmol), 2-methyl-quinoline-5-carboxylic acid (0.063g, 0.34 mmol), 1-[3-(dimethylamino)propyl]-3-ethylcarbodiimide hydrochloride (EDC) (0.064g, 0.34 mmol) and 1-hydroxybenzotriazole hydrate (HOBt) (0.01g, 0.07 mmol) in dichloromethane (4ml) was shaken at room temperature for 20h. The reaction mixture was washed with saturated aqueous sodium hydrogen carbonate (8ml) and the organic layer applied to a pre-packed 10g silica column. Elution with 0-100% ethyl acetate in pentane gradient then 0-10% methanol in ethyl acetate gradient gave the title compound as a colourless solid (0.037g, 23%). Mass spectrum (Electrospray LC/MS): Found 467 ($MH^+$). $C_{24}H_{27}{}^{79}BrN_4O$ requires 466.

The compounds of the Examples below were prepared from the appropriate piperidine using similar processes to that described in Examples 1 to 7.

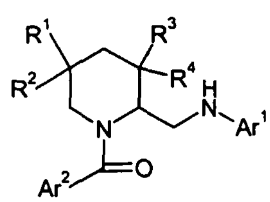

| Example | $R^1$ | $R^2$ | $R^3$ | $R^4$ | $Ar^1$ | $Ar^2$ | Amin e | Mass Spectrum (Electrospray LC/MS) |
|---|---|---|---|---|---|---|---|---|
| 8 | H | H | Me | Me | 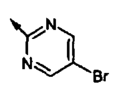 | 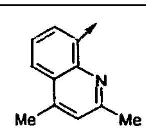 | D2 | Found 482 ($MH^+$). $C_{24}H_{28}{}^{79}BrN_5O$ requires 481. |
| 9 | H | H | Me | Me | 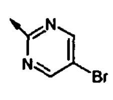 | 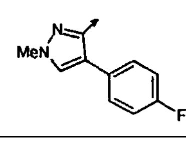 | D2 | Found 501 ($MH^+$). $C_{23}H_{26}{}^{79}BrFN_6O$ requires 500. |
| 10 | H | H | Me | Me | 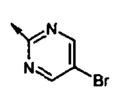 | 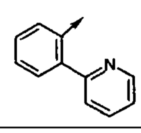 | D2 | Found 502 ($MNa^+$). $C_{24}H_{26}{}^{79}BrN_5O$ requires 479. |

(continued)

| Example | $R^1$ | $R^2$ | $R^3$ | $R^4$ | $Ar^1$ | $Ar^2$ | Amin e | Mass Spectrum (Electrospray LC/MS) |
|---|---|---|---|---|---|---|---|---|
| 11 | H | H | Me | Me | 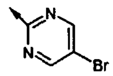 | 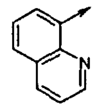 | D2a | Found 454 (MH+). $C_{22}H_{24}{}^{79}BrN_5O$ requires 453. |
| 12 | H | H | Me | Me | 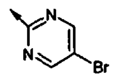 | 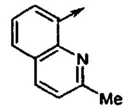 | D2a | Found 468 (MH+). 79 $C_{23}H_{26}{}^{79}BrN_5O$ requires 467. |
| 13 | H | H | Me | Me | 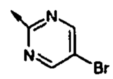 | 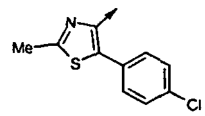 | D2a | Found 534 (MH+). $C_{23}H_{25}{}^{79}Br^{35}ClN_5$ OS requires 533. |
| 14 | Me | Me | H | H | 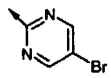 | 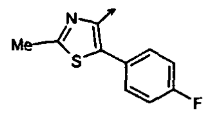 | D14 | Found 518 (MH+). $C_{23}H_{25}{}^{79}BrFN_5OS$ requires 517. |
| 15 | Me | Me | H | H | 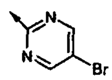 | 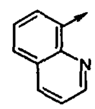 | D14 | Found 454 (MH+). $C_{22}H_{24}{}^{79}BrN_5O$ requires 453. |
| 16 | H | H | Me | Me | 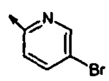 | 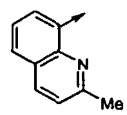 | D3 | Found 467 (MH+). $C_{24}H_{27}{}^{79}BrN_4O$ requires 466. |
| 17 | H | H | Me | Me | 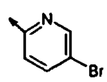 | 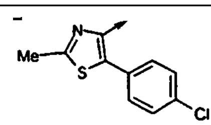 | D3 | Found 533 (MH+). $C_{24}H_{26}{}^{79}Br^{35}ClN_4$ OS requires 532. |
| 18 | H | H | Me | Me | 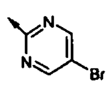 | 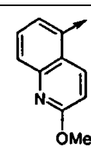 | D2a | Found 484 (MH+). $C_{23}H_{26}{}^{79}BrN_5O_2$ requires 483. |
| 19 | H | H | Me | Me | 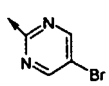 | 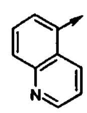 | D2a | Found 454 (MH+). $C_{22}H_{24}{}^{79}BrN_5O$ requires 453. |
| 20 | H | H | Me | Me | 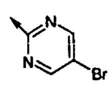 | 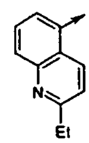 | D2a | Found 482 (MH+). $C_{24}H_{28}{}^{79}BrN_5O$ requires 481. |
| 21 | H | H | Me | Me | 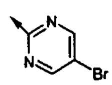 | 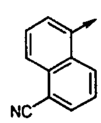 | D2a | Found 478 (MH+). $C_{24}H_{24}{}^{79}BrN_5O$ requires 477. |

(continued)

| Example | $R^1$ | $R^2$ | $R^3$ | $R^4$ | $Ar^1$ | $Ar^2$ | Amin e | Mass Spectrum (Electrospray LC/MS) |
|---|---|---|---|---|---|---|---|---|
| 22 | H | H | Me | Me | 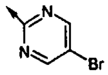 | 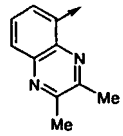 | D2a | Found 483 ($MH^+$). $C_{23}H_{27}{}^{79}BrN_6O$ requires 482. |
| 23 | H | H | Me | Me | 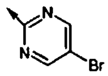 | 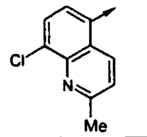 | D2a | Found 502 ($MH^+$). $C_{23}H_{25}\ {}^{79}BrClN_5\ O$ requires 501. |
| 24 | H | H | Me | Me | 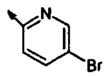 | 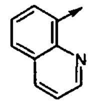 | D3 | Found 453 ($MH^+$). $C_{23}H_{25}{}^{79}BrN_4O$ requires 452. |
| 25 | H | H | Me | Me | 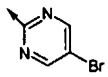 | 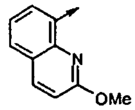 | D2a | Found 484 ($MH^+$). $C_{23}H_{26}{}^{79}BrN_5O_2$ requires 483. |
| 26 | H | H | Me | Me | 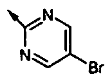 | 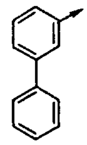 | D2a | Found 479 ($MH^+$). $C_{25}H_{27}{}^{79}BrN_4O$ requires 478. |
| 27 | H | H | Me | Me | 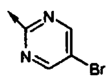 | 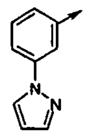 | D2a | Found 469 ($MH^+$). $C_{22}H_{25}{}^{79}BrN_6O$ requires 468. |
| 28 | H | H | Me | Me | 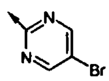 | 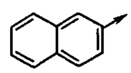 | D2a | Found 453 ($MH^+$). $C_{23}H_{25}{}^{79}BrN_4O$ requires 452. |
| 29 | H | H | Me | Me | 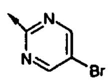 | 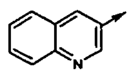 | D2a | Found 454 ($MH^+$). $C_{22}H_{24}{}^{79}BrN_5O$ requires 453. |
| 30 | H | H | Me | Me | 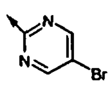 | 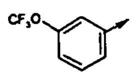 | D2a | Found 487 ($MH^+$). $C_{20}H_{22}{}^{79}BrF_3N_4O_2$ requires 486. |
| 31 | H | H | Me | Me | 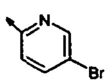 | 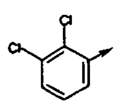 | D3a | Found 470 ($MH^+$). $C_{20}H_{22}{}^{79}Br^{35}Cl_2N_3\ O$ requires 469. |
| 32 | H | H | Me | Me | 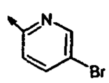 | 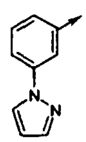 | D3a | Found 468 ($MH^+$). $C_{23}H_{26}{}^{79}BrN_5O$ requires 467. |
| 33 | H | H | Me | Me | 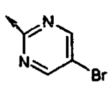 | 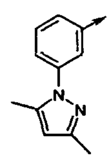 | D2a | Found 497 ($MH^+$). $C_{24}H_{29}{}^{79}BrN_6O$ requires 496. |

(continued)

| Example | $R^1$ | $R^2$ | $R^3$ | $R^4$ | $Ar^1$ | $Ar^2$ | Amin e | Mass Spectrum (Electrospray LC/MS) |
|---|---|---|---|---|---|---|---|---|
| 34 | H | H | Me | Me | 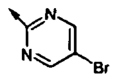 | 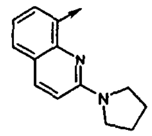 | D2a | Found 523 ($MH^+$). $C_{26}H_{31}{}^{79}BrN_6O$ requires 522. |
| 35 | Me | Me | H | H | 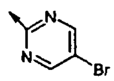 | 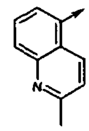 | D14b | Found 468 ($MH^+$). $C_{23}H_{26}{}^{79}BrN_5O$ requires 467. |
| 36 | H | H | Me | Me | 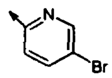 | 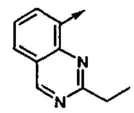 | D3a | Found 482 ($MH^+$). $C_{24}H_{28}{}^{79}BrN_5O$ requires 481. |
| 37 | H | H | Me | Me | 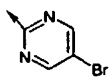 | 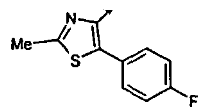 | D2a | Found 518 ($MH^+$). $C_{23}H_{25}{}^{79}BrFN_5OS$ requires 517. |
| 38 | H | H | Me | Me | 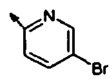 | 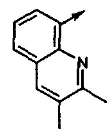 | D3a | Found 481 ($MH^+$) $C_{25}H_{29}{}^{79}BrN_4O$ requires 480. |
| 39 | H | H | Me | Me | 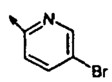 | 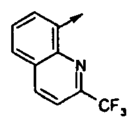 | D3a | Found 521 ($MH^+$). $C_{24}H_{24}{}^{79}BrF_3N_4O$ requires 520. |
| 40 | H | H | Me | Me | 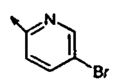 | 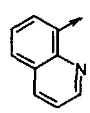 | D3a | Found 453 ($MH^+$). $C_{23}H_{25}{}^{79}BrN_4O$ requires 452. |
| 41 | H | H | Me | Me | 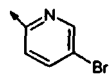 | 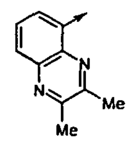 | D3a | Found 482 ($MH^+$). $C_{24}H_{28}{}^{79}BrN_5O$ requires 481. |
| 42 | H | H | Me | Me | 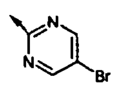 | 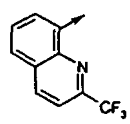 | D2a | Found 522 ($MH^+$). $C_{23}H_{23}{}^{79}BrF_3N_5O$ requires 521. |
| 43 | H | H | Me | Me | 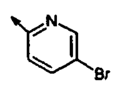 | 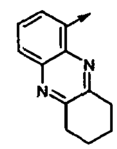 | D3a | Found 508 ($MH^+$). $C_{26}H_{30}{}^{79}BrN_5O$ requires 507. |

**Example 44: 1-{(S)-2-[(5-Bromo-pyrimidin-2-ylamino)-methyl]-3,3-dimethyl-piperidin-1-yl}-1-(2,3-dimethyl-quinolin-8-yl)-methanone**

To 2,3-dimethyl quinoline-8-carboxylic acid (0.5g, 2.5mmol) in dichloromethane (30ml) was added oxalyl chloride (0.48ml, 5.5mmol) dropwise, followed by dimethylformamide (1 drop). The resulting solution was stirred at ambient temperature for 1h. and then evaporated. The 2,3-dimethyl-8-quinolinecarbonyl chloride hydrochloride (0.64g, 100%) thus obtained as a brown solid was used without purification. To a solution of (+)-(5-bromo-pyrimidin-2-yl)-(3,3-dimethyl-piperidin-2-ylmethyl)-amine (D2a) (0.1g, 0.33mmol) and triethylamine (0.2ml, 1.44mmol) in dichloromethane (5ml) was added 2,3-dimethyl-8-quinolinecarbonyl chloride hydrochloride (0.087g, 0.34mmol). After 1h at ambient temperature, the reaction mixture was washed with saturated aqueous sodium hydrogencarbonate and the organic layer applied to a silica gel column. Elution with an ethyl acetate-pentane gradient afforded the title product (0.5g, 31%). Mass spectrum (Electrospray LC/MS): Found 482 ($MH^+$). $C_{24}H_{28}{}^{79}BrN_5O$ requires 481.

It is understood that the present invention covers all combinations of particular and preferred groups described herein above.

**Determination of Orexin-1 Receptor Antagonist Activity**

The orexin-1 receptor antagonist activity of the compounds of formula (I) was determined in accordance with the following experimental method.

**Experimental Method**

CHO-DG44 cells expressing the human orexin-1 receptor were grown in cell medium (MEM medium with Earl's salts) containing 2 mM L-Glutamine, 0.4 mg/mL G418 Sulphate from GIBCO BRL and 10% heat inactivated fetal calf serum from Gibco BRL. The cells were seeded at 20,000 cells/100 μl/well into 96-well black clear bottom sterile plates from Costar which had been pre-coated with 10 pg/well of poly-L-lysine from SIGMA. The seeded plates were incubated overnight at 37C in 5% $CO_2$.

Agonists were prepared as 1 mM stocks in water:DMSO (1:1). EC50 values (the concentration required to produce 50% maximal response) were estimated using 11 x half log unit dilutions (Biomek 2000, Beckman) in Tyrode's buffer containing probenecid (10 mM HEPES with 145mM Nad, 10mM glucose, 2.5 mM KCl, 1.5 mM $CaCl_2$, 1.2 mM $MgCl_2$ and 2.5mM probenecid; pH7.4). Antagonists were prepared as 10 mM stocks in DMSO (100%). Antagonist IC50 values (the concentration of compound needed to inhibit 50% of the agonist response) were determined against 3.0 nM human orexin-A using 11x half log unit dilutions in Tyrode's buffer containing 10% DMSO and probenecid.

On the day of assay 50 μl of cell medium containing probenecid (Sigma) and Fluo3AM (Texas Fluorescence Laboratories) was added (Quadra, Tomtec) to each well to give final concentrations of 2.5 mM and 4 μM, respectively. The 96-well plates were incubated for 60 min at 37C in 5% CO2. The loading solution containing dye was then aspirated and cells were washed with 4x150 μl Tyrode's buffer containing probenecid and 0.1% gelatin (Denley Cell Wash). The volume of buffer left in each well was 125 μl. Antagonist or buffer (25 μl) was added (Quadra) the cell plates gently shaken and incubated at 37C in 5% $CO_2$ for 30 minutes. Cell plates were then transferred to the Fluorescent Imaging Plate Reader (FLIPR, Molecular Devices) instrument. Prior to drug addition a single image of the cell plate was taken (signal test), to evaluate dye loading consistency. The run protocol used 60 images taken at 1 second intervals followed by a further 24 images at 5 second intervals. Agonists were added (by the FLIPR) after 20 seconds (during continuous reading). From each well, peak fluorescence was determined over the whole assay period and the mean of readings 1-19 inclusive was subtracted from this figure. The peak increase in fluorescence was plotted against compound concentration and iteratively curve fitted using a four parameter logistic fit (as described by Bowen and Jerman, *TiPS,* 1995, **16,** 413-417) to generate a concentration effect value. Antagonist Kb values were calculated using the equation:

$$Kb = IC50/(1+([3/EC50])$$

where EC50 was the potency of human orexin-A determined in the assay (in nM terms) and IC50 is expressed in molar terms.

Compounds of Examples tested according to this method had pKb values in the range 7.0 to 9.7 at the human cloned orexin-1 receptor.

The orexin-2 receptor antagonist activity of the compounds of formula (I) was determined in accordance with the following experimental method.

**Experimental Method**

CHO-DG44 cells expressing the human orexin-2 receptor were grown in cell medium (MEM medium with Earl's salts) containing 2 mM L-Glutamine, 0.4 mg/mL G418 Sulphate from GIBCO BRL and 10% heat inactivated fetal calf serum from Gibco BRL. The cells were seeded at 20,000 cells/100 μl/well into 96-well black clear bottom sterile plates from Costar which had been pre-coated with 10 μg/well of poly-L-lysine from SIGMA. The seeded plates were incubated overnight at 37C in 5% $CO_2$.

Agonists were prepared as 1 mM stocks in water:DMSO (1:1). EC50 values (the concentration required to produce 50% maximal response) were estimated using 11x half log unit dilutions (Biomek 2000, Beckman) in Tyrode's buffer containing probenecid (10 mM HEPES with 145mM NaCl, 10mM glucose, 2.5 mM KCl, 1.5 mM $CaCl_2$, 1.2 mM $MgCl_2$ and 2.5mM probenecid; pH7.4). Antagonists were prepared as 10 mM stocks in DMSO (100%). Antagonist IC50 values (the concentration of compound needed to inhibit 50% of the agonist response) were determined against 10.0 nM human orexin-A using 11 x half log unit dilutions in Tyrode's buffer containing 10% DMSO and probenecid.

On the day of assay 50 μl of cell medium containing probenecid (Sigma) and Fluo3AM (Texas Fluorescence Laboratories) was added (Quadra, Tomtec) to each well to give final concentrations of 2.5 mM and 4 μM, respectively. The 96-well plates were incubated for 60 min at 37C in 5% $CO_2$. The loading solution containing dye was then aspirated and cells were washed with 4x150 μl Tyrode's buffer containing probenecid and 0.1% gelatin (Denley Cell Wash). The volume of buffer left in each well was 125 μl. Antagonist or buffer (25 μl) was added (Quadra) the cell plates gently shaken and incubated at 37C in 5% $CO_2$ for 30 min. Cell plates were then transferred to the Fluorescent Imaging Plate Reader (FLIPR, Molecular Devices) instrument. Prior to drug addition a single image of the cell plate was taken (signal test), to evaluate dye loading consistency. The run protocol used 60 images taken at 1 second intervals followed by a further 24 images at 5 second intervals. Agonists were added (by the FLIPR) after 20 sec (during continuous reading). From each well, peak fluorescence was determined over the whole assay period and the mean of readings 1-19 inclusive was subtracted from this figure. The peak increase in fluorescence was plotted against compound concentration and iteratively curve fitted using a four parameter logistic fit (as described by Bowen and Jerman, *TiPS,* 1995, **16**, 413-417) to generate a concentration effect value. Antagonist Kb values were calculated using the equation:

$$Kb = IC50/(1+([3/EC50])$$

where EC50 was the potency of human orexin-A determined in the assay (in nM terms) and IC50 is expressed in molar terms.
Compounds of Examples tested according to this method had pKb values in the range <6.3 to 8.2 at the human cloned orexin-2 receptor.

## Claims

1. A compound of formula (I):

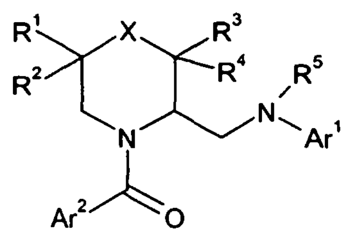

(I)

wherein:

X is O, $CR^7R^8$, NH or bond;
$R^1$ and $R^2$ are both hydrogen, both optionally substituted $(C_{1-4})$ alkyl, or are together with the carbon to which they are attached form a $(C_{3-6})$cycloalkyl ring or a 4- to 6- membered heterocyclyl ring.
$R^3$ and $R^4$ are both hydrogen, both optionally substituted $(C_{1-4})$ alkyl, or are together with the carbon to which they are attached form a $(C_{3-6})$cycloalkyl ring or a 4- to 6- membered heterocyclyl ring;
$R^7$ and $R^8$ are both hydrogen, both optionally substituted $(C_{1-4})$ alkyl, or are together with the carbon to which they are attached form a $(C_{3-6})$cycloalkyl ring or a 4- to 6- membered heterocyclyl ring;
provided that one pair of $R^1$ and $R^2$, $R^3$ and $R^4$, $R^7$ and $R^8$ are both optionally substituted $(C_{1-4})$ alkyl, or are together with the carbon to which they are attached form a $(C_{3-6})$cycloalkyl ring or a 4- to 6- membered heterocyclyl ring and the remaining groups are hydrogen;
$R^5$ is hydrogen, optionally substituted $(C_{1-4})$ alkyl, or optionally substituted $(C_{1-4})$alkylCO;

$Ar^1$ is an optionally substituted aryl, an optionally substituted mono or bicyclic heteroaryl group containing up to 3 heteroatoms selected from N, O and S;
$Ar^2$ represents phenyl or a 5- or 6-membered heterocyclyl group containing up to 3 heteroatoms selected from N, O and S, wherein the phenyl or heterocyclyl group is substituted by $R^6$ and further optional substituents; or $Ar^2$ represents an optionally substituted bicyclic aromatic or bicyclic heteroaromatic group containing up to 4 heteroatoms selected from N, O and S;
$R^6$ represents hydrogen, optionally substituted$(C_{1-4})$alkoxy, halo, cyano, optionally substituted$(C_{1-6})$alkyl, optionally substituted phenyl, or an optionally substituted 5- or 6-membered heterocyclyl group containing up to 4 heteroatoms selected from N, O and S;

or a pharmaceutically acceptable salt thereof.

**2.** A compound according to claim 1 wherein X is $CR^7R^8$.

**3.** A compound according to claim 1 or 2 wherein $R^1$, $R^2$, $R^7$ and $R^8$ are hydrogen when $R^3$ and $R^4$ are methyl or $R^3$, $R^4$, $R^7$ and $R^8$ are hydrogen when $R^1$ and $R^2$ are methyl.

**4.** A compound according to any one of claims 1 to 3 wherein $Ar^1$ is pyrimidinyl or pyridinyl.

**5.** A compound according to claim 4 wherein the pyrimidinyl or pyridinyl is substituted with halogen.

**6.** A compound according to claim 5 wherein the halogen is bromine.

**7.** A compound of formula (I) selected from the group consisting of:

(RS)-1-{2-[(5-Bromo-pyrimidin-2-ylamino)-methyl]-3,3-dimethyl-piperidin-1-yl} -1-[5-(4-fluoro-phenyl)-2-methyl-thiazol-4-yl]-methanone;
(RS)-1-{2-[(5-Bromo-pyrimidin-2-ylamino)-methyl]-3,3-dimethyl-piperidin-1-yl}-1-quinolin-8-yl-methanone;
1-{2-[(5-Bromo-pyrimidin-2-ylamino)-methyl]-3,3-dimethyl-piperidin-1-yl}-1-(2-methyl-quinolin-5-yl)-methanone;
(RS)-1-{2-[(5-Bromo-pyrimidin-2-ylamino)-methyl]-5,5-dimethyl-piperidin-1-yl}-1-(2-methyl-quinolin-5-yl)-methanone;
1- {2-[(5-Bromo-pyrimidin-2-ylamino)-methyl]-5,5-dimethyl-piperidin-1-yl}-1-(2-methyl-quinolin-5-yl)-methanone;
(RS)-1-{2-[(5-Bromo-pyridin-2-ylamino)-methyl]-3,3-dimethyl-piperidin-1-yl}-1-(2,3-dimethyl-quinoxalin-5-yl)-methanone;
(RS)-1-{2-[(5-Bromo-pyridin-2-ylamino)-methyl]-3,3-dimethyl-piperidin-1-yl}-1-(2-methyl-quinolin-5-yl)-methanone;
1- {(S)-2-[(5-Bromo-pyrimidin-2-ylamino)-methyl]-3,3-dimethyl-piperidin-1-yl}-1-(2, 3-dimethyl-quinolin-8-yl)-methanone;

or

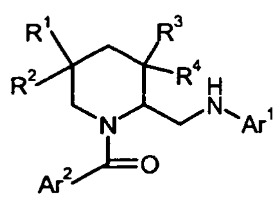

where:

| R¹ | R² | R³ | R⁴ | Ar¹ | Ar² |
|---|---|---|---|---|---|
| H | H | Me | Me | 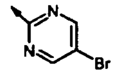 | 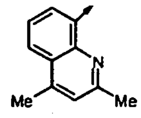 |
| H | H | Me | Me | 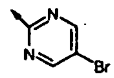 | 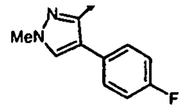 |
| H | H | Me | Me | 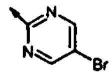 | 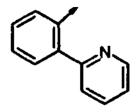 |
| H | H | Me | Me | 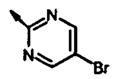 | 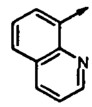 |
| H | H | Me | Me | 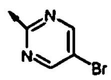 | 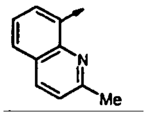 |
| H | H | Me | Me | 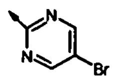 | 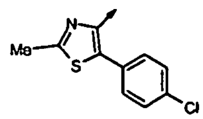 |
| Me | Me | H | H | 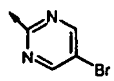 | 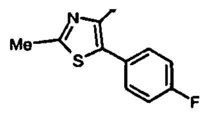 |
| Me | Me | H | H | 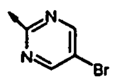 | 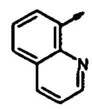 |
| H | H | Me | Me | 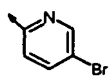 | 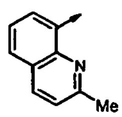 |
| H | H | Me | Me | 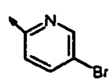 | 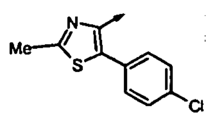 |
| H | H | Me | Me | 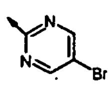 | 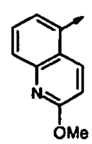 |
| H | H | Me | Me | 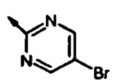 | 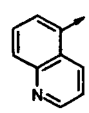 |
| H | H | Me | Me | 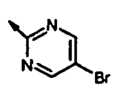 | 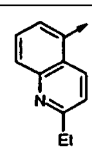 |

(continued)

| | | | | | |
|---|---|---|---|---|---|
| H | H | Me | Me | 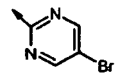 | 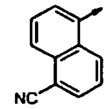 |
| H | H | Me | Me | 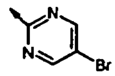 | 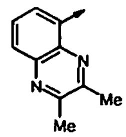 |
| H | H | Me | Me | 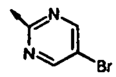 | 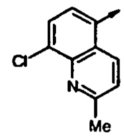 |
| H | H | Me | Me | 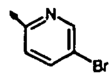 | 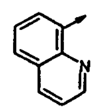 |
| H | H | Me | Me | 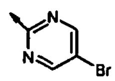 | 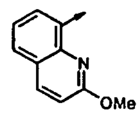 |
| H | H | Me | Me | 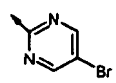 | 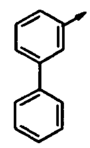 |
| H | H | Me | Me | 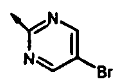 | 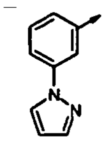 |
| H | H | Me | Me | 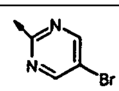 | 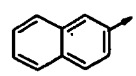 |
| H | H | Me | Me | 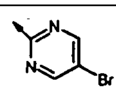 | 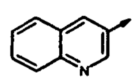 |
| H | H | Me | Me | 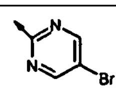 | 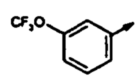 |
| H | H | Me | Me | 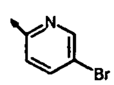 | 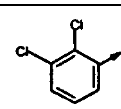 |
| H | H | Me | Me | 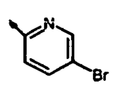 | 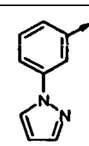 |
| H | H | Me | Me | 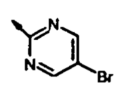 | 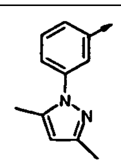 |

(continued)

| | | | | | |
|---|---|---|---|---|---|
| H | H | Me | Me | 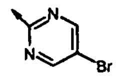 | 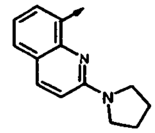 |
| H | H | Me | Me | 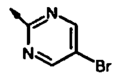 | 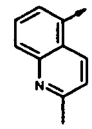 |
| H | H | Me | Me | 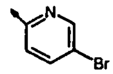 | 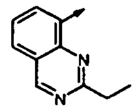 |
| H | H | Me | Me | 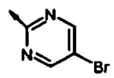 | 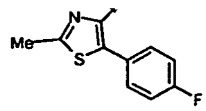 |
| H | H | Me | Me | 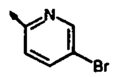 | 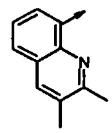 |
| H | H | Me | Me | 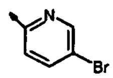 | 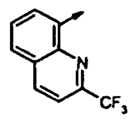 |
| H | H | Me | Me | 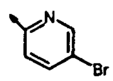 | 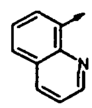 |
| H | H | Me | Me | 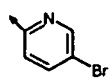 | 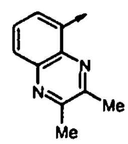 |
| H | H | Me | Me | 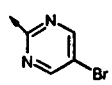 | 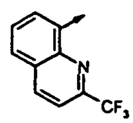 |
| H | H | Me | Me | 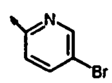 | 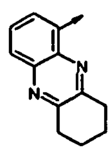 |

or a pharmaceutically acceptable salt of any one thereof.

**8.** A pharmaceutical composition comprising a compound of formula (I) as defined in any one of claims 1 to 7, or a pharmaceutically acceptable salt thereof, and a pharmaceutically acceptable carrier.

**9.** Use of a compound of formula (I) as defined in any one of claims 1 to 7, or a pharmaceutically acceptable derivative thereof, in the manufacture of a medicament for the treatment or prophylaxis of diseases or disorders where an antagonist of a human Orexin receptor is required.

**10.** Use of a compound of formula (I), or a pharmaceutically acceptable derivative thereof, in the manufacture of a medicament for the treatment of obesity.

**11.** Use of a compound of formula (I), or a pharmaceutically acceptable derivative thereof, in the manufacture of a medicament for the treatment of sleep disorders.

**Patentansprüche**

**1.** Verbindung der Formel (I):

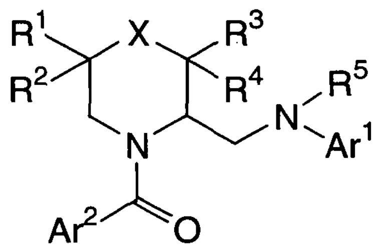

(I)

worin:

X O, $CR^7R^8$, NH oder eine Bindung ist;
$R^1$ und $R^2$ beide Wasserstoff sind, beide gegebenenfalls substituiertes $C_{1-4}$-Alkyl sind oder zusammen mit dem Kohlenstoff, an den sie gebunden sind, einen $C_{3-6}$-Cycloalkylring oder einen 4- bis 6-gliedrigen Heterocyclylring bilden;
$R^3$ und $R^4$ beide Wasserstoff sind, beide gegebenenfalls substituiertes $C_{1-4}$-Alkyl sind oder zusammen mit dem Kohlenstoff, an den sie gebunden sind, einen $C_{3-6}$-Cycloalkylring oder einen 4- bis 6-gliedrigen Heterocyclylring bilden;
$R^7$ und $R^8$ beide Wasserstoff sind, beide gegebenenfalls substituiertes $C_{1-4}$-Alkyl sind oder zusammen mit dem Kohlenstoff, an den sie gebunden sind, einen $C_{3-6}$-Cycloalkylring oder einen 4- bis 6-gliedrigen Heterocyclylring bilden;
mit der Maßgabe, daß ein Paar aus $R^1$ und $R^2$, $R^3$ und $R^4$, $R^7$ und $R^8$ beide gegebenenfalls substituiertes $C_{1-4}$-Alkyl sind oder zusammen mit dem Kohlenstoff, an den sie gebunden sind, einen $C_{3-6}$-Cycloalkylring oder einen 4-bis 6-gliedrigen Heterocyclylring bilden und die verbleibenden Gruppen Wasserstoff sind;
$R^5$ Wasserstoff, gegebenenfalls substituiertes $C_{1-4}$-Alkyl oder gegebenenfalls substituiertes $C_{1-4}$-Alkyl-CO ist;
$Ar^1$ gegebenenfalls substituiertes Aryl oder eine gegebenenfalls substituierte mono- oder bicyclische Heteroarylgruppe ist, die bis zu 3 Heteroatome enthält, die aus N, O und S ausgewählt sind;
$Ar^2$ Phenyl oder eine 5- oder 6-gliedrige Heterocyclylgruppe darstellt, die bis zu 3 Heteroatome enthält, die aus N, O und S ausgewählt sind, worin die Phenyl- oder Heterocyclylgruppe mit $R^6$ und weiteren optionalen Substituenten substituiert ist; oder $Ar^2$ eine gegebenenfalls substituierte bicyclische aromatische oder bicyclische heteroaromatische Gruppe darstellt, die bis zu 4 Heteroatome enthält, die aus N, O und S ausgewählt sind;
$R^6$ Wasserstoff, gegebenenfalls substituiertes $C_{1-4}$-Alkoxy, Halogen, Cyano, gegebenenfalls substituiertes $C_{1-6}$-Alkyl, gegebenenfalls substituiertes Phenyl oder eine gegebenenfalls substituierte 5- oder 6-gliedrige Heterocyclylgruppe darstellt, die bis zu 4 Heteroatome enthält, die aus N, O und S ausgewählt sind;

oder ein pharmazeutisch akzeptables Salz davon.

**2.** Verbindung gemäß Anspruch 1, worin X $CR^7R^8$ ist.

**3.** Verbindung gemäß Anspruch 1 oder 2, worin $R^1$, $R^2$, $R^7$ und $R^8$ Wasserstoff sind, wenn $R^3$ und $R^4$ Methyl sind, oder $R^3$, $R^4$, $R^7$ und $R^8$ Wasserstoff sind, wenn $R^1$ und $R^2$ Methyl sind.

**4.** Verbindung gemäß einem der Ansprüche 1 bis 3, worin $Ar^1$ Pyrimidinyl oder Pyridinyl ist.

**5.** Verbindung gemäß Anspruch 4, worin das Pyrimidinyl oder Pyridinyl mit Halogen substituiert ist.

**6.** Verbindung gemäß Anspruch 5, worin das Halogen Brom ist.

**7.** Verbindung der Formel (I), die aus der Gruppe ausgewählt ist, die aus:

(RS)-1-{2-[(5-Brom-pyrimidin-2-ylamino)-methyl]-3,3-dimethyl-piperidin-1-yl}-1-[5-(4-fluorphenyl)-2-methylthiazol-4-yl]-methanon,
(RS)-1-{2-[(5-Brom-pyrimidin-2-ylamino)-methyl]-3,3-dimethyl-piperidin-1-yl}-1-chinolin-8-yl-methanon,
1-{2-[(5-Brom-pyrimidin-2-ylamino)-methyl]-3,3-dimethyl-piperidin-1-yl}-1-(2-methyl-chinolin-5-yl)-methanon,
(RS)-1-{2-[(5-Brom-pyrimidin-2-ylamino)-methyl]-5,5-dimethyl-piperidin-1-yl}-1-(2-methyl-chinolin-5-yl)-methanon,
1-{2-[(5-Brom-pyrimidin-2-ylamino)-methyl]-5,5-dimethyl-piperidin-1-yl}-1-(2-methyl-chinolin-5-yl)-methanon,
(RS)-1-{2-[(5-Brom-pyridin-2-ylamino)-methyl]-3,3-dimethyl-piperidin-1-yl}-1-(2,3-dimethyl-chinoxalin-5-yl)-methanon,
(RS)-1-{2-[(5-Brom-pyridin-2-ylamino)-methyl]-3,3-dimethyl-piperidin-1-yl}-1-(2-methyl-chinolin-5-yl)-methanon,
1-{(S)-2-[(5-Brom-pyrimidin-2-ylamino)-methyl]-3,3-dimethyl-piperidin-1-yl}-1-(2,3-dimethyl-chinolin-8-yl)-methanon
oder

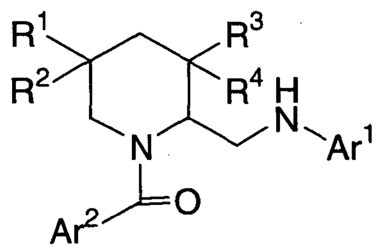

besteht, worin gilt:

| $R^1$ | $R^2$ | $R^3$ | $R^4$ | $Ar^1$ | $Ar^2$ |
|---|---|---|---|---|---|
| H | H | Me | Me | 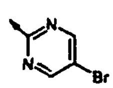 | 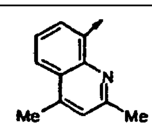 |
| H | H | Me | Me | 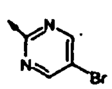 | 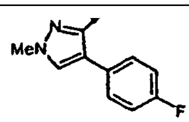 |
| H | H | Me | Me | 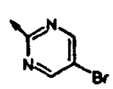 | 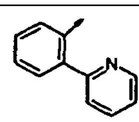 |
| H | H | Me | Me | 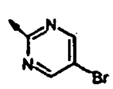 | 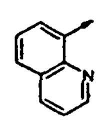 |
| H | H | Me | Me | 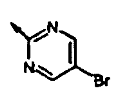 | 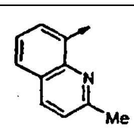 |
| H | H | Me | Me | 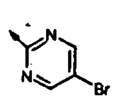 | 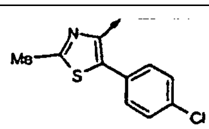 |

(fortgesetzt)

| $R^1$ | $R^2$ | $R^3$ | $R^4$ | $Ar^1$ | $Ar^2$ |
|---|---|---|---|---|---|
| Me | Me | H | H | 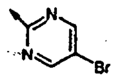 | 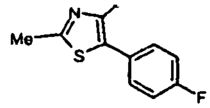 |
| Me | Me | H | H | 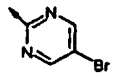 | 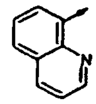 |
| H | H | Me | Me | 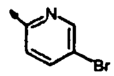 | 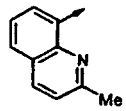 |
| H | H | Me | Me | 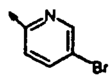 | 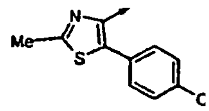 |
| H | H | Me | Me | 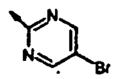 | 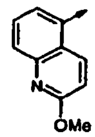 |
| H | H | Me | Me | 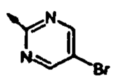 | 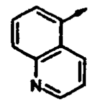 |
| H | H | Me | Me | 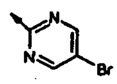 | 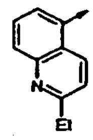 |
| H | H | Me | Me | 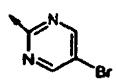 | 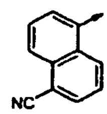 |
| H | H | Me | Me | 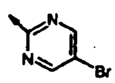 | 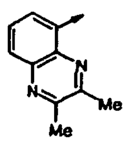 |
| H | H | Me | Me | 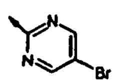 | 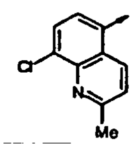 |
| H | H | Me | Me | 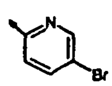 | 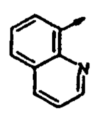 |
| H | H | Me | Me | 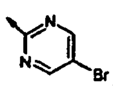 | 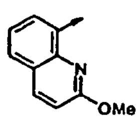 |

(fortgesetzt)

| R1 | R2 | R3 | R4 | Ar1 | Ar2 |
|---|---|---|---|---|---|
| H | H | Me | Me | 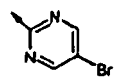 | 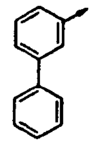 |
| H | H | Me | Me | 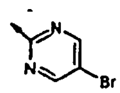 | 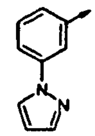 |
| H | H | Me | Me | 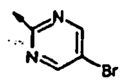 | 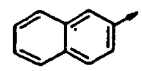 |
| H | H | Me | Me | 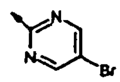 | 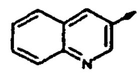 |
| H | H | Me | Me | 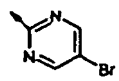 | 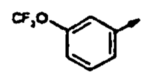 |
| H | H | Me | Me | 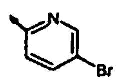 | 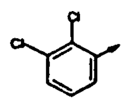 |
| H | H | Me | Me | 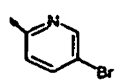 | 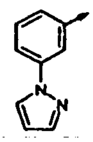 |
| H | H | Me | Me | 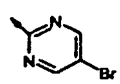 | 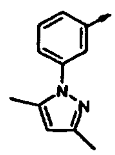 |
| H | H | Me | Me | 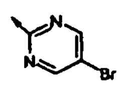 | 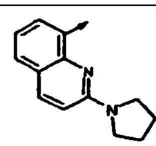 |
| Me | Me | H | H | 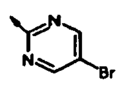 | 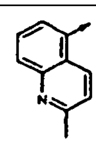 |
| H | H | Me | Me | 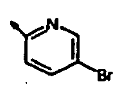 | 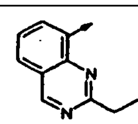 |
| H | H | Me | Me | 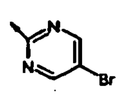 | 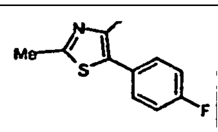 |

(fortgesetzt)

| $R^1$ | $R^2$ | $R^3$ | $R^4$ | $Ar^1$ | $Ar^2$ |
|---|---|---|---|---|---|
| H | H | Me | Me | 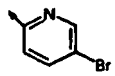 | 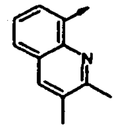 |
| H | H | Me | Me | 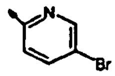 | 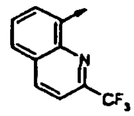 |
| H | H | Me | Me | 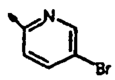 | 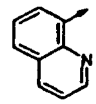 |
| H | H | Me | Me | 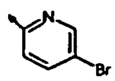 | 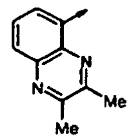 |
| H | H | Me | Me | 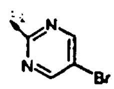 | 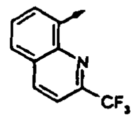 |
| H | H | Me | Me | 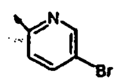 | 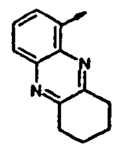 |

oder ein pharmazeutisch akzeptables Salz von jeder davon.

**8.** Pharmazeutische Zusammensetzung, die eine Verbindung der Formel (I) wie in einem der Ansprüche 1 bis 7 definiert oder ein pharmazeutisch akzeptables Salz davon und einen pharmazeutisch akzeptablen Träger umfaßt.

**9.** Verwendung einer Verbindung der Formel (I) wie in einem der Ansprüche 1 bis 7 definiert oder eines pharmazeutisch akzeptablen Derivats davon in der Herstellung eines Medikaments zur Behandlung oder Prophylaxe von Krankheiten oder Störungen, in denen ein Antagonist eines humanen Orexinrezeptors erforderlich ist.

**10.** Verwendung einer Verbindung der Formel (I) oder eines pharmazeutisch akzeptablen Derivats davon in der Herstellung eines Medikaments zur Behandlung von Fettsucht.

**11.** Verwendung einer Verbindung der Formel (I) oder eines pharmazeutisch akzeptablen Derivats davon in der Herstellung eines Medikaments zur Behandlung von Schlafstörungen.

## Revendications

**1.** Composé de formule (I)

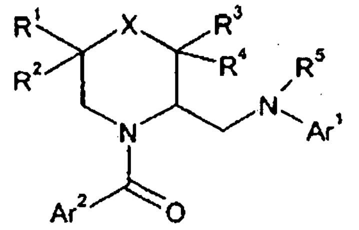

(I)

dans laquelle :

- X représente O, un groupe $CR^7R^8$, NH ou une liaison ;
- $R^1$ et $R^2$ représentent tous deux l'hydrogène, tous deux un groupe alkyle $C_{1-4}$ éventuellement substitué ou bien ils forment en association avec l'atome de carbone auquel ils sont attachés, un cycle cycloalkyle $C_{3-6}$ ou un cycle hétérocyclyle de 4 à 6 atomes ;
- $R^3$ et $R^4$ représentent tous deux l'hydrogène, tous deux un groupe alkyle $C_{1-4}$ éventuellement substitué ou bien ils forment, en association avec l'atome de carbone auquel ils sont attachés, un cycle cycloalkyle $C_{3-6}$ ou un cycle hétérocyclyle de 4 à 6 atomes ;
- $R^7$ et $R^8$ représentent tous deux l'hydrogène, tous deux un groupe alkyle $C_{1-4}$ éventuellement substitué ou bien ils forment, en association avec l'atome de carbone auquel ils sont attachés, un cycle cycloalkyle $C_{3-6}$ ou un cycle hétérocyclyle de 4 à 6 atomes ;

à la condition que les groupes d'une paire parmi $R^1$ et $R^2$, $R^3$ et $R^4$, $R^7$ et $R^8$ représentent tous deux un groupe alkyle $C_{1-4}$ éventuellement substitué, ou que, en association avec l'atome de carbone auquel ils sont attachés, ils forment un cycle cycloalkyle $C_{3-6}$ ou un cycle hétérocyclyle de 4 à 6 atomes, et que les groupes restants représentent l'hydrogène ;

- $R^5$ représente l'hydrogène, un groupe alkyle $C_{1-4}$ éventuellement substitué, ou un groupe alkyl($C_{1-4}$)-CO éventuellement substitué ;
- $Ar^1$ représente un groupe aryle éventuellement substitué, un groupe hétéroaryle mono- ou bi-cyclique contenant jusqu'à 3 hétéro-atomes choisis parmi N, O et S.
- $Ar^2$ représente un groupe phényle ou un groupe hétérocyclyle à 5 ou 6 atomes contenant jusqu'à 3 hétéroatomes choisis parmi N, O et S, où le groupe phényle ou hétérocyclyle est substitué par $R^6$ et d'autres substituants éventuels ; ou bien $Ar^2$ représente un groupe aromatique bicyclique éventuellement substitué, ou un groupe hétéro-aromatique bicyclique éventuellement substitué contenant jusqu'à 4 hétéroatomes choisis parmi N, O et S ;
- $R^6$ représente l'hydrogène, un groupe alcoxy $C_{1-4}$ éventuellement substitué, halo, cyano, alkyle $C_{1-6}$ éventuellement substitué, phényle éventuellement substitué ou hétérocyclyle contenant jusqu'à 4 hétéroatomes choisis parmi N, O et S ;

ou un sel pharmaceutiquement acceptable, de celui-ci.

**2.** Composé selon la revendication 1, dans lequel X représente $CR^7R^8$.

**3.** Composé selon la revendication 1 ou la revendication 2, dans lequel $R^1$, $R^2$ $R^7$ et $R^8$ représentent l'hydrogène lorsque $R^3$ et $R^4$ représentent un groupe méthyle, ou bien $R^3$, $R^4$, $R^7$ et $R^8$ représentent l'hydrogène lorsque $R^1$ et $R^2$ représentent un groupe méthyle.

**4.** Composé selon l'une quelconque des revendications 1 à 3, dans lequel $Ar^1$ représente un groupe pyrimidinyle ou pyridinyle.

**5.** Composé selon la revendication 4, dans lequel le groupe pyrimidinyle ou pyridinyle est substitué par un halogène.

**6.** Composé selon la revendication 5, dans lequel l'halogène est le brome.

**7.** Composé de formule (I) choisi au sein du groupe consistant en de la :

- (RS)-1-{2-[(S-Bromo-pyrimidin-2-ylamino)méthyl]-3,3-diméthyl-pipéridin-1-yl}-1-[5-(4-fluorophényl)-2-méthyl-thiazol-4-yl]méthanone ;
- (RS)-1-{2-[(5-Bromo-pyrimidin-2-ylamino)méthyl]-3,3-diméthyl-pipéridin-1-yl}-1-quinoléin-8-yl-méthanone ;
- 1-{2-[(5-Bromo-pyrimidin-2-ylamino)méthyl]-3,3-diméthyl-pipéridin-1-yl}-1-(2-méthylquinoléin-5-yl) méthanone ;
- (RS)-1-{2-[(5-Bromo-pyrimidin-2-ylamino)méthyl]-5,5-diméthyl-pipéridin-1-yl}-1-(2-méthylquinoléin-5-yl) méthanone ;
- 1-{2-[(5-Bromo-pyrimidin-2-ylamino)méthyl]-5,5-diméthyl-pipéridin-1-yl}-1-(2-méthylquinoléin-5-yl) méthanone ;
- (RS)-1-{2-[(5-Bromo-pyridin-2-ylamino)méthyl]-3,3-diméthyl-pipéridin-1-yl}-1-(2,3-diméthylquinoxalin-5-yl) méthanone ;
- (RS)-1-{2-[(5-Bromo-pyridin-2-ylamino)méthyl]-3,3-diméthyl-pipéridin-1-yl}-1-(2-méthylquinoléin-5-yl) méthanone ;
- 1-{(S)-2-[(5-Bromo-pyrimidin-2-ylamino)méthyl]-3,3-diméthyl-pipéridin-1-yl}-1-(2,3-diméthylquinoléin-8-yl) méthanone ;

ou

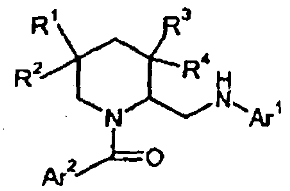

où

| $R^1$ | $R^2$ | $R^3$ | $R^4$ | $Ar^1$ | $Ar^2$ |
|---|---|---|---|---|---|
| H | H | Me | Me | 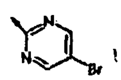 | 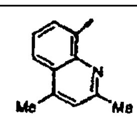 |
| H | H | Me | Me | 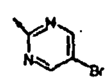 | 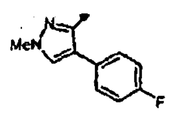 |
| H | H | Me | Me | 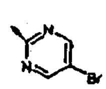 | 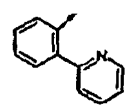 |
| H | H | Me | Me | 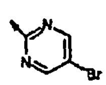 | 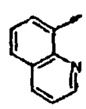 |
| H | H | Me | Me | 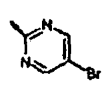 | 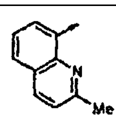 |
| H | H | Me | Me | 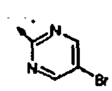 | 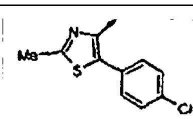 |
| Me | Me | H | H | 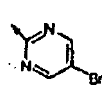 | 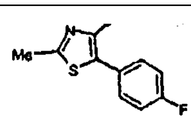 |

(suite)

| $R^1$ | $R^2$ | $R^3$ | $R^4$ | $Ar^1$ | $Ar^2$ |
|---|---|---|---|---|---|
| Me | Me | H | H | 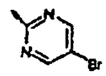 | 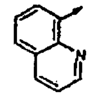 |
| H | H | Me | Me | 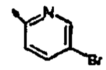 | 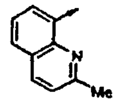 |
| H | H | Me | Me | 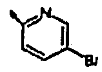 | 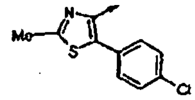 |
| H | H | Me | Me | 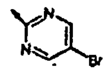 | 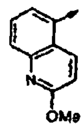 |
| H | H | Me | Me | 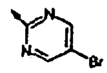 | 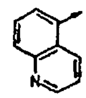 |
| H | H | Me | Me | 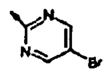 | 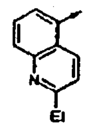 |
| H | H | Me | Me | 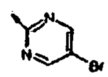 | 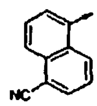 |
| H | H | Me | Me | 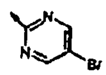 | 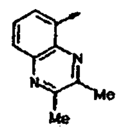 |
| H | H | Me | Me | 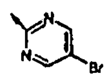 | 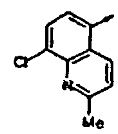 |
| H | H | Me | Me | 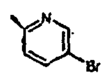 | 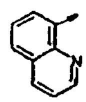 |
| H | H | Me | Me | 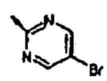 | 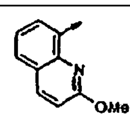 |
| H | H | Me | Me | 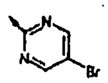 | 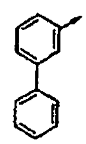 |
| H | H | Me | Me | 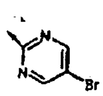 | 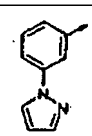 |

(suite)

| R1 | R2 | R3 | R4 | Ar1 | Ar2 |
|---|---|---|---|---|---|
| H | H | Me | Me | 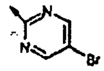 | 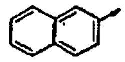 |
| H | H | Me | Me | 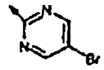 | 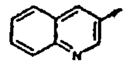 |
| H | H | Me | Me | 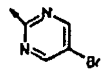 | 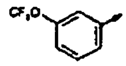 |
| H | H | Me | Me | 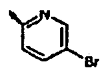 | 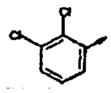 |
| H | H | Me | Me | 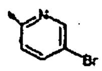 | 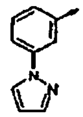 |
| H | H | Me | Me | 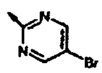 | 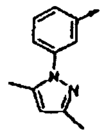 |
| H | H | Me | Me | 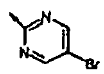 | 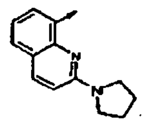 |
| Me | Me | H | H | 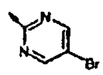 | 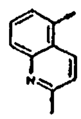 |
| H | H | Me | Me | 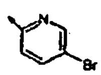 | 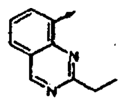 |
| H | H | Me | Me | 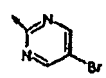 | 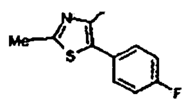 |
| H | H | Me | Me | 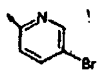 | 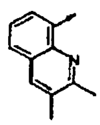 |
| H | H | Me | Me | 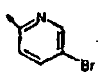 | 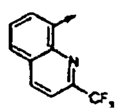 |
| H | H | Me | Me | 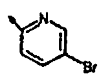 | 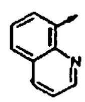 |

(suite)

| $R^1$ | $R^2$ | $R^3$ | $R^4$ | $Ar^1$ | $Ar^2$ |
|---|---|---|---|---|---|
| H | H | Me | Me | 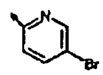 | 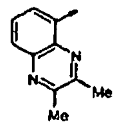 |
| H | H | Me | Me | 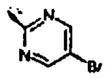 | 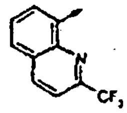 |
| H | H | Me | Me | 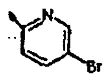 | 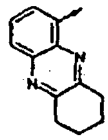 |

ou un sel pharmaceutiquement acceptable de l'un quelconque d'entre eux.

**8.** Composition pharmaceutique comprenant un composé de formule (I) selon l'une quelconque des revendications 1 à 7, ou un sel pharmaceutiquement acceptable de celui-ci, et un véhicule pharmaceutiquement acceptable.

**9.** Utilisation d'un composé de formule (I) selon l'une quelconque des revendications 1 à 7, ou d'un dérivé pharmaceutiquement acceptable de celui-ci, dans la fabrication d'un médicament pour le traitement ou la prophylaxie de maladies ou de troubles pour lesquels il est requis un antagoniste d'un récepteur humain d'Orexine.

**10.** Utilisation d'un composé de formule (I) ou d'un dérivé pharmaceutiquement acceptable de celui-ci, dans la fabrication d'un médicament pour le traitement de l'obésité.

**11.** Utilisation d'un composé de formule (I) ou d'un dérivé pharmaceutiquement acceptable de celui-ci, dans la fabrication d'un médicament pour le traitement de troubles du sommeil.